# EUROPEAN PATENT SPECIFICATION

(11) **EP 4 243 993 B1**
(45) Date of publication and mention of the grant of the patent: **07.10.2026**
(21) Application number: 21891341.6
(22) Date of filing: 12.11.2021
(51) Int. Cl.: A61M 11/02, A61M 11/00, A61M 15/00, B05B 11/06

(54) **DEVICES FOR DELIVERING A SUBSTANCE TO A BODY CAVITY**
VORRICHTUNGEN ZUR ABGABE EINER SUBSTANZ IN EINE KÖRPERHÖHLE
DISPOSITIFS D'ADMINISTRATION D'UNE SUBSTANCE DANS UNE CAVITÉ CORPORELLE

(30) Priority: 14.11.2020 US 202063113834 P; 25.06.2021 US 202117358707
(43) Date of publication of application: 20.09.2023
(73) Proprietor: AptarGroup, Inc., Crystal Lake, IL 60014-9695 (US)
(72) Inventor: SHAHAF, Daniel, Emek Ha-Yarden (IL); SHICHOR, Iris, 30900 Zichron Ya'akov (IL); HADAR, Liron Shavit, 3508425 Haifa (IL); KAUFMAN, Lia, 3092098 Zichron Ya'akov (IL)
(74) Representative: Vossius & Partner Patentanwälte Rechtsanwälte mbB
(86) International application number: PCT/IB2021/060520
(87) International publication number: WO 2022/101851

(56) References cited:
- EP-B1- 1 023 098
- WO-A1-2020/154182
- WO-A1-99/58180
- US-A- 4 017 007
- US-A1- 2003 127 533
- US-A1- 2020 197 633
- US-A1- 2020 306 463
- US-B1- 6 186 141
- US-B1- 6 398 074

## Description

### CROSS REFERENCE TO RELATED APPLICATIONS

The present application claims the benefit of priority to U.S. Provisional Application No. 63/113,834 dated November 14, 2020 and U.S. Application No. 17/358,707, filed June 25, 2021.

U.S. Application No. 17/358,707 is a Continuation-in-Part of U.S. application No. 16/809,994, filed on March 5, 2020, which is a Continuation-in-Part of U.S. application No. 15/982,996 filed on May 17, 2018, which is a Continuation-in-Part of U.S. application No. 14/733,143 filed on Jun. 8, 2015, which claims the benefit of and priority to U.S. Provisional Application No. 62/117,986 filed on Feb. 19, 2015, and U.S. Provisional Application No. 62/077,246 filed on Nov. 9, 2014. U.S. application No. 15/982,996 claims further priority to U.S. Provisional Application No. 62/526,386 filed on Jun. 29, 2017.

U.S. application No. 16/809,994 is also a Continuation-in-Part of U.S. Application No. 16/810,096 filed March 5, 2020, which is a Continuation-in-Part of U.S. Application No. 15/982,630 filed on May 17, 2018, which is a Continuation-in-Part of U.S. application No. 14/733,143 filed on Jun. 8, 2015, which claims the benefit of and priority to U.S. Provisional Application No. 62/117,986 filed on Feb. 19, 2015, and U.S. Provisional Application No. 62/077,246 filed on Nov. 9, 2014. U.S. application No. 15/982,630 further claims priority to U.S. Provisional Application No. 62/507,816 filed on May 18, 2017.

Further, U.S. application No. 16/809,994 is a Continuation-in-Part of U.S. application No. 14/433,048 filed on Apr. 2, 2015, which is a National Phase Entry of PCT/IL2014/050752 filed on Aug. 21, 2014, which claims the benefit of and priority to U.S. Provisional Application No. 61/868,614 filed on Aug. 22, 2013, and U.S. Provisional Application No. 61/868,627 filed on Aug. 22, 2013, and further priority to German Application No. 2020131057150 filed on Dec. 16, 2013.

Additionally, U.S. Application No. 17/358,707 claims priority to and the benefit of US. Provisional Application No. 63/113,834 filed on November 14, 2020.

U.S. Application No. 17/358,707 is a Continuation-in-Part of International Application No. PCT/IB2021/051834 filed on March 4, 2021.

### FIELD OF THE INVENTION

The present invention generally pertains to a system for delivering aerosolized substance to a natural orifice of the body.

### BACKGROUND OF THE INVENTION

Blow-Fill-Seal (BFS) technology is a manufacturing technique used to produce small (0.1ml) and large (and up to 500 ml) liquid-filled containers. The basic concept of blow-fill-seal and form-fill-seal (referred to interchangeably hereinafter as BFS) is that a container is formed, filled and sealed in a continuous process without human intervention in a sterile or aseptic enclosed area. Thus, this technology can be used to sterile or aseptically packaging and manufacturing of pharmaceutical liquid dosage forms. Furthermore, this can be applied to any drug container (produced by any production methods) with a laminate at the bottom thereof, which can be pierced (or break) so as to deliver the medicament.

There are several ways of manufacturing. According to one method, the processes begun as pharmaceutical grade plastic resin is vertically heat-extruded through a circular throat to form a hanging tube (parison). This extruded tube is then enclosed within a two-part mold and the tube is cut above the mold. The mold is zone, or a sterile filling space, where filling needles (mandrels) are lowered and used to inflate the plastic to form a container within the mold. Following formation of the container, the mandrel is used to fill the container with the liquid. Following filling, the mandrels are retracted and a secondary top mold seals the container. All actions take place within the sterile enclosed area, a sterile shrouded chamber within the machine. The product can then be discharged to a non-sterile area for labeling, packaging and distribution.

BFS technology reduces personnel intervention, making it a more robust method for aseptic preparation of sterile pharmaceuticals. BFS is used for the filling of vials for parenteral preparations and infusion, eye drops, and inhalation products. Generally, the containers are made of polyethylene and polypropylene or any plastic resin.

It is therefore a long felt need to provide a system which provides an efficient delivery of a substance to a target site from such BFS containers, provides sufficient material to the target site, and ensures reproducibility.

US 6186141 B1 discloses a device for dispensing a unit dose of substance. The device comprises a nozzle assembly including a container housing a unit dose of the substance, and an outer body to which the nozzle assembly is releasably attached. The body includes a system actuable to effect discharge of the substance from the nozzle assembly and actuating means operably by a user to effect discharge. The discharge system comprises a spring-loaded air piston actuable to inject air into the container to cause discharge of the substance. A driving spring for the air piston is loaded with spring energy by the action of installing the nozzle assembly on the body, the driving spring thereafter being held in a cocked state until operating of the actuating means by the user to effect release of the spring energy to cause discharge.

US 2020/197633 A1 discloses a BFS/FFS system for delivering substances within a body cavity, the system comprising a vial comprising said substances, the vial selected from a pierceable container, a blow-fill-seal and a form-fill-seal, having fluid inlet and a fluid discharging outlet, configured for placement in proximity to said body cavity; said fluid inlet configured to interface in a sealable manner a puncturing member, configured to, upon coupling to said fluid inlet, piercing the same, thereby providing said substances in a fluid communication, via a valve, with a chamber configured to accept pressurized fluid; said valve being commutable from a CLOSE to an OPEN configuration; wherein at said OPEN configuration said pressurized fluid flows from said chamber, via said fluid inlet, entrains said substances, and erupts via said fluid discharging outlet to within said body cavity.

Further drug delivery devices of the prior art are disclosed for example in WO 99/58180 A1, WO 2020/154182 A1, US 4 017 007 A, US 6 398 074 B1, and US 2003/127533 A1.

### SUMMARY OF THE INVENTION

It is one object of the present invention to provide a device for delivering either one or more substances within at least one body cavity as defined in the appended independent claim 1. Preferred embodiments of the invention are matter of the dependent claims. According to the current technology, a device for delivering either one or more substances within at least one body cavity, comprises:
(i) at least one nosepiece comprising at least one capsule comprising V_{sub} [ml or mg] of said substances; said capsule having at least one fluid inlet port of diameter Dᵢₙ [mm] and at least one fluid discharging outlet of diameter Dₒᵤₜ [mm], configured for placement in proximity to said body cavity;
(ii) at least one base in communication with said at least one nosepiece, said at least one base comprises at least one chamber configured to confine compressed and pressurized fluid at volume V_{PF} [ml] and pressure P_{PF} [barg];
(iii) at least one hollow puncturing member; said at least one hollow puncturing member is characterized by at least one sharp end and a second end in fluid communication with said at least one base; said first and second ends are fluidly interconnected by at least one hollow tube; said fluid inlet port of said capsule is configured by means of size and shape to interface in a sealable manner with said one sharp end of said at least one puncturing member;
wherein actuation of said base configured to enable piercing of said capsule to provide said fluid inlet port, by means of said sharp end of said puncturing member to enables the pressurized fluid to exit said chamber and entrain through said hollow tube of said hollow puncturing member to said capsule, entrain said substance and deliver the same to said body cavity.

According to an aspect of the technology, said chamber is made of material being high barrier film.

According to an aspect of the technology, said high barrier film is high barrier Aluminum film.

According to an aspect of the technology, actuation of said base is pressing on the same.

According to an aspect of the technology, said second end of said at least one hollow puncturing member and said chamber are formed as a single unit, such that the upper surface of said chamber is integrated with said second end of said at least one hollow puncturing member.

According to an aspect of the technology, said sharp end of said at least one hollow puncturing member comprises at least one orifice throughout which said pressurized fluid enters said capsule.

According to an aspect of the technology, the device additionally comprises at least one sealing septum, adapted to, prior to actuation of said base, circumference and seal said at least one orifice in said sharp end of said at least one hollow puncturing member.

According to an aspect of the technology, upon actuation of said device, said at least one sealing septum is removed to enable exit of said pressurized fluid throughout said at least one orifice in said at least one hollow puncturing member.

According to an aspect of the technology, upon actuation of said device, said based is pressed and said hollow puncturing member is pushed towards said nosepiece such that the bottom most part of said nosepiece removes said sealing septum from circumferencing said at least one orifice in said sharp end of said at least one hollow puncturing member.

According to an aspect of the technology, said sharp end of said hollow puncturing member comprises at least one orifice, such that, once said device is actuated, said pressurized fluid enters said capsule through said at least one orifice said hollow puncturing member.

According to an aspect of the technology, said chamber comprises at least one orifice throughout which said pressurized fluid exits said chamber.

According to an aspect of the technology, said second end of said hollow puncturing member seals said at least one orifice of said chamber, prior to actuation of said device.

According to an aspect of the technology, upon actuation of said device said sealing of said at least one orifice of said chamber is removed, such that said pressurized fluid exits through said at least one orifice of said chamber into said hollow tube of said hollow puncturing member.

According to an aspect of the technology, said sharp end of said hollow puncturing member comprises at least one orifice, such that, once said device is actuated, said pressurized fluid enters said capsule through said at least one orifice said hollow puncturing member.

According to an aspect of the technology, said nosepiece comprises at least one nosepiece cover configured by means of size and shape to cover, in a sealable manner, at least partially said nosepiece

According to an aspect of the technology, said nosepiece cover and said nosepiece are coupled to each other.

According to an aspect of the technology, said coupling between nosepiece cover and said nosepiece said is reversible.

According to an aspect of the technology, said nosepiece comprises at least one port throughout which said at least one substance exits said device, such that said nosepiece cover seals said at least one port and removal thereof removes said seal.

According to an aspect of the technology, said capsule is selected from a group consisting of pierceable container, a blow-fill-seal and a form-fill-seal and any combination thereof.

According to an aspect of the technology, said at least one chamber is a container adapted to hold said pressured fluid at said P_{PF} for prolong periods of time.

According to an aspect of the technology, capsule is made of at least one material selected from a group consisting of high- or low-density polyethylene, high- or low-density polypropylene, any plastic resin, glass and any combination thereof.

According to an aspect of the technology, said volume V_{PF} [ml] of said pressurized fluid at pressure P_{PF} [barg] is released from said chamber within a short period of time, <500 milliseconds (dT), via said fluid inlet port, entrains said substances, erupts via said fluid discharging outlet port into said body cavity, such that the release time of said V_{sub} [ml or mg] of said substances and said V_{PF} [ml] of said pressurized fluid, dTᵣₑₗₑₐₛₑ is less than 500 milliseconds.

According to an aspect of the technology, said device is configured to deliver said V_{sub} substance and V_{PF} pressurized fluid through said fluid discharging outlet of diameter D [mm] wherein at least one of the following is held true:
a. V_{PF} is in a range of 1 to 50 ml;
b. V_{sub} is in a range of about 0.01 to about 7 ml;
c. Dᵢₙ and/or Dₒᵤₜ is in a range of 0.2 to 6 mm;
d. P_{PF} is in a range of about 0 to about 10 barg;
e. said pressure velocity is greater than 0.001 barg /ms;
f. said pressure velocity is greater than 0.01 barg /ms;
g. said volume rate dVsub/dT or dV_{sub}/dTᵣₑₗₑₐₛₑ is greater than 0.0001 ml /ms;
h. said volume rate dV_{sub}/dT or dV_{sub}/dTᵣₑₗₑₐₛₑ is greater than 0.001 ml /ms;
i. said volume rate dV_{PF}/dT or dV_{PF}/dTᵣₑₗₑₐₛₑ is greater than 0.001 ml /ms;
j. said volume rate dV_{PF}/dT or dV_{PF}/dTᵣₑₗₑₐₛₑ is greater than 0.01 ml /ms;
k. any combination thereof.

According to an aspect of the technology, at least one of the following is true:
a. said body cavity is selected from a group consisting of nasal cavity, the mouth, the throat, an ear, the eye, the vagina, the rectum, the urethra, and any combination thereof.
b. said pressurized gas is selected from a group consisting of air, nitrogen, oxygen, carbon dioxide, helium, neon, xenon, nitric oxide and any combination thereof;
c. during dispensing of said at least one substance, a mixture of said predetermined volume V_{gas} [ml] of said pressurized gas with said predetermined volume V_{sub} [ml or mg] of said substance entrained within it forms a plume of aerosol; said aerosol having a predetermined distribution, said distribution being either homogeneous or heterogeneous, said heterogeneous distribution is selected from a group consisting of: an arbitrary distribution, a distribution in which the density of said at least one substance within said mixture follows a predetermined pattern, and any combination thereof; characteristics of said aerosol selected from a group consisting of: particle size, particle shape, particle distribution, and any combination thereof, are determinable from characteristics of said device selected from a group consisting of: said predetermined volume of said pressurized gas, said predetermined volume of said substance, said predetermined pressure of said pressurized gas, said predetermined orifice size, and any combination thereof;
d. at least one of said substance is selected from a group consisting of a gas, a liquid, a powder, an aerosol, a slurry, a gel, a suspension and any combination thereof;
e. at least one said substance is stored under either an inert atmosphere or under vacuum to prevent reactions during storage;
f. a dose-response curve is substantially linear for brain concentration of said substance when administered nasally via said device; and
g. a dose-response curve for brain concentration having a fit selected from a group consisting of logarithmic, parabolic, exponential, sigmoid, power-low, and any combination thereof; of said substance when administered nasally via said device.

According to an aspect of the technology, said nosepiece cover configured to provide an air-tight closure for said port, said port cover slidable along said device, rotatable around said device, rotatable around a hinge on the exterior of said device and any combination thereof.

According to an aspect of the technology, said pressurized fluid entrains said substance in a pulsed manner, such that a plurality of potions V_{PF} erupts via said fluid discharging outlet to within said body cavity.

According to an aspect of the technology, the device further comprises a safety latch, adapted to prevent accidental operation of said device.

According to an aspect of the technology, said substance is selected from a group consisting of proteins; stem-cells; cells, organs, portions, extracts, and isolations thereof; macro-molecules; RNA or other genes and proteins-encoding materials; neurotransmitters; receptor antagonists; hormones; Ketamine; Baqsimi product commercially available by Lilly (US); Glucagon; substrates to treat one of eth followings: anaphylaxis, Parkinson, seizures and opioid overdose; epinephrine; atropine; metoclopramide; commercially available Naloxone or Narcan products; Esketamine (Spravato); Radicava [edaravone]; Ingrezza [valbenazine]; Austedo [deutetrabenazine]; Ocrevus [ocrelizumab]; Xadago [safinamide]; Spinraza [nusinersen]; Zinbryta [daclizumab]; Nuplazid [pimavanserin]; Aristada [aripiprazole lauroxil]; Vraylar [cariprazine]; Rexulti [brexpiprazole]; Aptiom [eslicarbazepine acetate]; Vizamyl [flutemetamol F18 injection]; Brintellix [vortioxetine]; Tecfidera [dimethyl fumarate]; Dotarem [gadoterate meglumine]; Antibody mediated brain targeting drug delivery including aducanumab, gantenerumab, bapineuzumab, solanezumab, ofatumumab CD20, BIIB033, LCN2, HMGB1; insulin; oxytocin; orexin-A; leptin; benzodiazepine, midazolam; naloxone; perillyl alcohol; camptothecin; phytochemicals including curcumin and chrysin; nucleotides; olanzapine; risperidone; Venlafaxin; GDF-5; zonisamide; ropinirole; plant-originated and synthetically-produced terpenes and cannabinoids, including THC and CBD; valproric acid; rivastigmine; estradiol; topiramate or an equivalent preparation comprising CAS No. 97240-79-4; MFSD2 or MFSD2A or sodium-dependent lysophosphatidylcholine symporter, midazolam; naloxone; perillyl alcohol; camptothecin; phytochemicals including curcumin and chrysin; nucleotides; olanzapine; risperidone; Venlafaxin; GDF-5; zonisamide; ropinirole; plant-originated and synthetically-produced terpenes and cannabinoids, including THC and CBD; valproric acid; rivastigmine; estradiol; topiramate or an equivalent preparation comprising CAS No. 97240-79-4; MFSD2 or MFSD2A or sodium-dependent lysophosphatidylcholine symporter; and any esters, salts, derivatives, mixtures, combinations thereof, with or without a carrier, liposomes, lyophilic or water-miscible solvents, surfactants, cells, cells fractions, at a therapeutically effective concentration.

According to an aspect of the technology, said capsule is a hollow tube characterized by at least two ends interconnect to each other, at least one of which is positioned proximal to said chamber.

According to an aspect of the technology, said capsule comprises at least one spherical element positioned at at least one of said ends, adapted to seal said capsule and prevent leakage of said at least one substrate therefrom.

According to an aspect of the technology, said capsule comprises two spherical elements, each of which is disposed at each of said ends, such that said at least one substrate is position therebetween.

According to an aspect of the technology, said two spherical elements, once said pressurized fluid exits said chamber, are adapted to mix said at least one substrate and said at least one substrate.

According to an aspect of the technology, said capsule comprises at least one membrane positioned at at least one of said ends, adapted to seal said capsule and prevent leakage of said at least one substrate therefrom.

According to an aspect of the technology, said capsule comprises two membranes, each of which is disposed at each of said ends, such that said at least one substrate is position therebetween.

According to an aspect of the technology, said capsule comprises at least one duckbill valve positioned at at least one of said ends, adapted to seal said capsule and prevent leakage of said at least one substrate therefrom.

According to an aspect of the technology, said capsule comprises two duckbill valves, each of which is disposed at each of said ends, such that said at least one substrate is position therebetween.

According to an aspect of the technology, said capsule comprises at least one spherical element, membrane, unidirectional valve, duckbill valve and any combination thereof.

Further, there is disclosed, but not independently claimed a method for delivering either one or more substances within at least one body cavity, characterized by steps of
a. providing:
   i. at least one nosepiece comprising at least one capsule comprising V_{sub} [ml or mg] of said substances; said capsule having at least one fluid inlet port of diameter Dᵢₙ [mm] and at least one fluid discharging outlet of diameter Dₒᵤₜ [mm], configured for placement in proximity to said body cavity;
   ii. at least one base in communication with said at least one nosepiece, said at least one base comprises at least one chamber configured to confine compressed and pressurized fluid at volume V_{PF} [ml] and pressure P_{PF} [barg];
   iii. at least one hollow puncturing member; said at least one hollow puncturing member is characterized by at least one sharp end and a second end in fluid communication with said at least one base; said first and second ends are fluidly interconnected by at least one hollow tube; said fluid inlet port of said capsule is configured by means of size and shape to interface in a sealable manner with said one end of said at least one puncturing member;
b. actuating said base thereby piercing of said capsule in said nosepiece, by means of said at least one puncturing member.

According to an aspect of the technology, in said method said chamber is made of material being high barrier film.

According to an aspect of the technology, in said method said high barrier film is high barrier Aluminum film.

According to an aspect of the technology, in said method step (b) of actuating said base additionally comprises pressing said base.

According to an aspect of the technology, in said method step (b) of actuating said base enables the pressurized fluid to exit said chamber and entrain through said hollow tube of said hollow puncturing member to said capsule, entrain said substance and deliver the same to said body cavity.

According to an aspect of the technology, in said method step (b) of actuating said base results in releasing said volume V_{PF} [ml] of said pressurized fluid at pressure P_{PF} [barg] within a short period of time, <500 milliseconds (dT); out of said chamber, via said fluid inlet thereby entraining said substances and erupting via said fluid discharging outlet into said body cavity, such that the release time of said V_{sub} [ml or mg] of said substances and said V_{PF} [ml] of said pressurized fluid, dTᵣₑₗₑₐₛₑ is less than 500 milliseconds.

According to an aspect of the technology, in said method said second end of said at least one hollow puncturing member and said chamber are formed as a single unit, such that the upper surface of said chamber is integrated with said second end of said at least one hollow puncturing member.

According to an aspect of the technology, in said method said sharp end of said at least one hollow puncturing member comprises at least one orifice throughout which said pressurized fluid enters said capsule.

According to an aspect of the technology, in said method said device additionally comprises at least one sealing septum, adapted to, prior to actuation of said base, circumference and seal said at least one orifice in said sharp end of said at least one hollow puncturing member.

According to an aspect of the technology, in said method upon actuation of said device, said at least one sealing septum is removed to enable exit of said pressurized fluid throughout said at least one orifice in said at least one hollow puncturing member.

According to an aspect of the technology, in said method upon actuation of said device, said based is pressed and said hollow puncturing member is pushed towards said nosepiece such that the bottom most part of said nosepiece removes said sealing septum from circumferencing said at least one orifice in said sharp end of said at least one hollow puncturing member.

According to an aspect of the technology, in said method said sharp end of said hollow puncturing member comprises at least one orifice, such that, once said device is actuated, said pressurized fluid enters said capsule through said at least one orifice said hollow puncturing member

According to an aspect of the technology, in said method said chamber comprises at least one orifice throughout which said pressurized fluid exits said chamber.

According to an aspect of the technology, in said method said second end of said hollow puncturing member seals said at least one orifice of said chamber, prior to actuation of said device.

According to an aspect of the technology, in said method upon actuation of said device said sealing of said at least one orifice of said chamber is removed, such that said pressurized fluid exits through said at least one orifice of said chamber into said hollow tube of said hollow puncturing member.

According to an aspect of the technology, in said method said sharp end of said hollow puncturing member comprises at least one orifice, such that, once said device is actuated, said pressurized fluid enters said capsule through said at least one orifice said hollow puncturing member.

According to an aspect of the technology, in said method said nosepiece comprises at least one nosepiece cover configured by means of size and shape to cover, in a sealable manner, at least partially said nosepiece

According to an aspect of the technology, in said method said nosepiece comprises at least one nosepiece cover and said nosepiece are coupled to each other.

According to an aspect of the technology, in said method said coupling between nosepiece cover and said nosepiece said is reversible.

According to an aspect of the technology, in said method removal of said nosepiece cover results in piercing said capsule to provide said fluid discharging outlet.

According to an aspect of the technology, in said method removal of said nosepiece cover is obtained by at least one action selected from a group consisting of sliding said nosepiece cover along said device, rotating said nosepiece cover around said device, rotating said nosepiece cover around a hinge on the exterior of said device and any combination thereof.

According to an aspect of the technology, in said method said nosepiece cover comprises at least one nosepiece puncturing member adapted to pierce said capsule to enable said fluid discharging outlet.

According to an aspect of the technology, in said method said nosepiece comprises at least one port throughout which said at least one substance exits said device, such that said nosepiece cover seals said at least one port and removal thereof removes said seal.

According to an aspect of the technology, in said method said capsule is selected from a group consisting of pierceable container, a blow-fill-seal and a form-fill-seal and any combination thereof. According to an aspect of the technology, in said method said at least one chamber is a container adapted to hold said pressured fluid at said P_{PF} for prolong periods of time.

According to an aspect of the technology, in said method capsule is made of at least one material selected from a group consisting of high- or low-density polyethylene, high- or low-density polypropylene, any plastic resin, glass and any combination thereof.

According to an aspect of the technology, in said method said device is configured to deliver said V_{sub} substance and V_{PF} pressurized fluid through said fluid discharging outlet of diameter D [mm] wherein at least one of the following is held true:
a. V_{PF} is in a range of 1 to 50 ml;
b. V_{sub} is in a range of about 0.01 to about 7 ml;
c. Dᵢₙ and/or Dₒᵤₜ is in a range of 0.2 to 6 mm;
d. P_{PF} is in a range of about 0 to about 10 barg;
e. said pressure velocity is greater than 0.001 barg /ms;
f. said pressure velocity is greater than 0.01 barg /ms;
g. said volume rate dVsub/dT or dVsub /dTᵣₑₗₑₐₛₑ is greater than 0.0001 ml /ms;
h. said volume rate dV_{sub}/dT or dV_{sub} /dTᵣₑₗₑₐₛₑ is greater than 0.001 ml /ms;
i. said volume rate dV_{PF}/dT or dV_{PF}/dTᵣₑₗₑₐₛₑ is greater than 0.001 ml /ms;
j. said volume rate dV_{PF}/dT or dV_{PF}/dTᵣₑₗₑₐₛₑ is greater than 0.01 ml /ms; and
k. any combination thereof.

According to an aspect of the technology, said method additionally comprises at least one of the following steps
a. selecting said body cavity from a group consisting of a nasal cavity, the mouth, the throat, an ear, the eye, the vagina, the rectum, the urethra, and any combination thereof;
b. selecting said gas from a group consisting of: air, nitrogen, oxygen, carbon dioxide, helium, neon, xenon, nitric oxide and any combination thereof;
c. dispensing said at least one substance, and during said step of dispensing, forming a plume of aerosol with predetermined distribution from a mixture of said predetermined volume V_{gas} [ml] of said pressurized gas and said predetermined volume V_{sub} [ml or mg] entrained within it; selecting said predetermined distribution from a group consisting of: a homogeneous distribution, a heterogeneous distribution; selecting said heterogeneous distribution from a group consisting of: an arbitrary distribution, a distribution in which the density of said at least one substance within said mixture follows a predetermined pattern, and any combination thereof; selecting characteristics of said aerosol from a group consisting of: particle size, particle shape, particle distribution, and any combination thereof, are determinable from characteristics of said device selected from a group consisting of: said predetermined volume of said pressurized gas, said predetermined volume of said substance, said predetermined pressure of said pressurized gas, said predetermined orifice size, and any combination thereof;
d. selecting said substance from a group consisting of: a gas, a liquid, a powder, a slurry, a gel, a suspension, and any combination thereof;
e. storing at least one said substance under either an inert atmosphere or under vacuum, thereby preventing reactions during storage; and
f. characterizing a dose-response curve for brain concentration of said substance to be of substantially linear form; and
g. a dose-response curve for brain concentration having a fit selected from a group consisting of logarithmic, parabolic, exponential, sigmoid, power-low, and any combination thereof; of said substance when administered nasally via said device.

According to an aspect of the technology, in said method said substance is selected from a group consisting of proteins; stem-cells; cells, organs, portions, extracts, and isolations thereof; macro-molecules; RNA or other genes and proteins-encoding materials; neurotransmitters; receptor antagonists; hormones; Ketamine; Baqsimi product commercially available by Lilly (US); Glucagon; substrates to treat one of eth followings: anaphylaxis, Parkinson, seizures and opioid overdose; epinephrine; atropine; metoclopramide; commercially available Naloxone or Narcan products; Esketamine (Spravato); Radicava [edaravone]; Ingrezza [valbenazine]; Austedo [deutetrabenazine]; Ocrevus [ocrelizumab]; Xadago [safinamide]; Spinraza [nusinersen]; Zinbryta [daclizumab]; Nuplazid [pimavanserin]; Aristada [aripiprazole lauroxil]; Vraylar [cariprazine]; Rexulti [brexpiprazole]; Aptiom [eslicarbazepine acetate]; Vizamyl [flutemetamol F18 injection]; Brintellix [vortioxetine]; Tecfidera [dimethyl fumarate]; Dotarem [gadoterate meglumine]; Antibody mediated brain targeting drug delivery including aducanumab, gantenerumab, bapineuzumab, solanezumab, ofatumumab CD20, BIIB033, LCN2, HMGB1; insulin; oxytocin; orexin-A; leptin; benzodiazepine i.e. midazolam; naloxone; perillyl alcohol; camptothecin; phytochemicals including curcumin and chrysin; nucleotides; olanzapine; risperidone; Venlafaxin; GDF-5; zonisamide; ropinirole; plant-originated and synthetically-produced terpenes and cannabinoids, including THC and CBD; valproric acid; rivastigmine; estradiol; topiramate or an equivalent preparation comprising CAS No. 97240-79-4; MFSD2 or MFSD2A or sodium-dependent lysophosphatidylcholine symporter; and any esters, salts, derivatives, mixtures, combinations thereof, with or without a carrier, liposomes, lyophilic or water-miscible solvents, surfactants, cells, cells fractions, at a therapeutically effective concentration.

According to an aspect of the technology, in said method said puncturing member comprises a plurality of holes throughout which said pressurized fluid exits said chamber and entrains said substance, after activation of said activation mechanism.

According to an aspect of the technology, in said method said capsule is a hollow tube characterized by at least two ends interconnect to each other, at least one of which is positioned proximal to said chamber.

According to an aspect of the technology, in said method said capsule comprises at least one spherical element positioned at at least one of said ends, adapted to seal said capsule and prevent leakage of said at least one substrate therefrom.

According to an aspect of the technology, in said method said capsule comprises two spherical elements, each of which is disposed at each of said ends, such that said at least one substrate is position therebetween.

According to an aspect of the technology, in said method said two spherical elements, once said pressurized fluid exits said chamber, are adapted to mix said at least one substrate and said at least one substrate.

According to an aspect of the technology, in said method said capsule comprises at least one membrane positioned at at least one of said ends, adapted to seal said capsule and prevent leakage of said at least one substrate therefrom.

According to an aspect of the technology, in said method said capsule comprises two membranes, each of which is disposed at each of said ends, such that said at least one substrate is position therebetween.

According to an aspect of the technology, in said method said capsule comprises at least one duckbill valve positioned at at least one of said ends, adapted to seal said capsule and prevent leakage of said at least one substrate therefrom.

According to an aspect of the technology, in said method said capsule comprises two duckbill valves, each of which is disposed at each of said ends, such that said at least one substrate is position therebetween.

According to an aspect of the technology, in said method said capsule comprises at least one spherical element, membrane, unidirectional valve, duckbill valve and any combination thereof.

According to an aspect of the technology, in said method said at least one hollow puncturing member comprises at least one orifice throughout which said pressurized fluid enters said hollow puncturing member.

According to an aspect of the technology, in said method, once said device is actuated, said pressurized fluid exit said chamber and enters said hollow puncturing member through said at least one orifice.

According to an aspect of the technology, in said method said at least one hollow puncturing member comprises at least one orifice throughout which said pressurized fluid enters said hollow puncturing member.

According to an aspect of the technology, in said method, once said device is actuated, said pressurized fluid exit said chamber and enters said hollow puncturing member through said at least one orifice.

According to an aspect of the technology, in said method said chamber is comprised of at least one material selected from a group consisting of high- or low-density polyethylene, high- or low-density polypropylene, any plastic resin, glass, plastics, Aluminum and any combination thereof.

According to an aspect of the technology, in said method said chamber is comprised of at least one material selected from a group consisting of high- or low-density polyethylene, high- or low-density polypropylene, any plastic resin, glass, plastics, Aluminum and any combination thereof.

In accordance with an aspect of the technology, there is disclosed a device for delivering either one or more substances within at least one body cavity. The device is characterized by at least one pierceable vial comprising V_{sub} [ml or mg] of the substances; the vial having at least one fluid inlet port of diameter Dᵢₙ [mm]and at least one fluid discharging outlet port of diameter Dₒᵤₜ [mm], configured for placement in proximity to the body cavity; the fluid inlet port configured by means of size and shape to interface with at least one puncturing member, configured to, upon coupling to the fluid inlet port, piercing the same, thereby providing the substances in a fluid communication, with at least one chamber configured to accept pressurized fluid at volume V_{PF} [ml] and pressure P_{PF} [barg]; the pressurized fluid flows from the chamber, via the fluid inlet port, entrains the substances, erupts via the fluid discharging outlet port to within the body cavity in the form of aerosol, such that the release time of the V_{sub} [ml or mg] of the substances and the V_{PF} [ml] of the pressurized fluid, dTᵣₑₗₑₐₛₑ is less than 500 milliseconds; In this device, the following held: V_{PF} is in a range of 1 to 50 ml; V_{sub} is in a range of about 0.01 to about 7 ml or 0.1 mg to 7 g; P_{PF} is in a range of about 0 to about 10 barg; further wherein at least one of the following is being held true: Dᵢₙ or Dₒᵤₜ are in a range of 0.2 to 6 mm; the pressure velocity is greater than 0.001 barg /ms; the pressure velocity is greater than 0.01 barg /ms; the volume rate dV_{sub}/dTᵣₑₗₑₐₛₑ is greater than 0.0001 ml /ms; the volume rate dV_{sub}/dTᵣₑₗₑₐₛₑ is greater than 0.001 ml /ms; the volume rate dV_{PF}/dTᵣₑₗₑₐₛₑ is greater than 0.001 ml /ms; the volume rate dV_{PF}/dTᵣₑₗₑₐₛₑ is greater than 0.01 ml /ms; any combination thereof. The vial is further selected from a group consisting of a pierceable container, a blow-fill-seal and a form-fill-seal and any combination thereof.

According to an aspect of the technology, the vial comprises a cap adapted to seal the vial, such that removal thereof provides fluid communication between the vial and the body cavity through the fluid discharging outlet port.

According to an aspect of the technology, the at least one puncturing member is adapted to pierce the vial be means of a screw mechanism, such that rotation of the nosepiece cover along the screw mechanism in the base enables the pierce of the fluid inlet port in the vial by means of the puncturing member.

According to an aspect of the technology, at least one of the following is true: (a) The body cavity is selected from a group consisting of nasal cavity, the mouth, the throat, an ear, the eye, the vagina, the rectum, the urethra, and any combination thereof. (b) The pressurized gas is selected from a group consisting of air, nitrogen, oxygen, carbon dioxide, helium, neon, xenon, nitric oxide and any combination thereof. (c) During dispensing of the at least one substance, a mixture of the predetermined volume V_{gas} [ml] of the pressurized gas with the predetermined volume V_{sub} [ml or mg] of the substance entrained within it forms a plume of aerosol; the aerosol having a predetermined distribution, the distribution being either homogeneous or heterogeneous, the heterogeneous distribution is selected from a group consisting of: an arbitrary distribution, a distribution in which the density of the at least one substance within the mixture follows a predetermined pattern, and any combination thereof; characteristics of the aerosol selected from a group consisting of: particle size, particle shape, particle distribution, and any combination thereof, are determinable from characteristics of the device selected from a group consisting of: the predetermined volume of the pressurized gas, the predetermined volume of the substance, the predetermined pressure of the pressurized gas, the predetermined orifice size, and any combination thereof. (d) At least one the substance is selected from a group consisting of a gas, a liquid, a powder, an aerosol, a slurry, a gel, a suspension and any combination thereof. (e) The least one the substance is stored under either an inert atmosphere or under vacuum to prevent reactions during storage. (f) A dose-response curve is substantially linear for brain concentration of the substance when administered nasally via the device; and (g) A dose-response curve for brain concentration having a fit selected from a group consisting of logarithmic, parabolic, exponential, sigmoid, power-low, and any combination thereof; of the substance when administered nasally via the device.

According to an aspect of the technology, the vial is a capsule having a main longitudinal axis, the capsule comprising a number *n* of compartments, the capsule configured to contain the predetermined volume V_{sub} [ml or mg] of the at least one substance, the volume V_{sub} [ml or mg] of the at least one substance containable in at least one of the *n* compartments; at least one of the following being true: the number *n* of the compartments is an integer greater than or equal to 1; at least one the compartment has cross-section with shape selected from a group consisting of: wedge shaped, circular, oval, elliptical, polygonal, annular, and any combination thereof; for the number n of compartments being an integer greater than 1, at least two the compartments have different volumes; for the number n of compartments being an integer greater than 1, at least two the compartments have the same volume; for the number *n* of compartments being an integer greater than 1, at least two the compartments have different cross-sectional areas; for the number *n* of compartments being an integer greater than 1, at least two the compartments have the same cross-sectional area; for the number *n* of compartments being an integer greater than 1, at least two the compartments contain different substances; for the number *n* of compartments being an integer greater than 1, at least two the compartments contain the same substance; for the number *n* of compartments being an integer greater than 1, at least two the compartments are disposed coaxially around the main longitudinal axis of the capsule; for the number *n* of compartments being an integer greater than 1, at least two the compartments are disposed sequentially along the main longitudinal axis of the capsule; for the number *n* of compartments greater than 1, the plurality of substances mix during the dispensing; and for the number *n* of compartments greater than 1, the plurality of substances react during the dispensing.

According to an aspect of the technology, the vial comprises a port fluidly connectable to the exterior of the device, the port configured such that the at least one substance is insertable into the chamber via the port.

According to an aspect of the technology, the device comprises a port cover configured to provide an air-tight closure for the port, the port cover slidable along the device, rotatable around the device, rotatable around a hinge on the exterior of the device and any combination thereof.

According to an aspect of the technology, the pressurized fluid entrains the substance in a pulsed manner, such that a plurality of potions V_{PF} erupts via the fluid discharging outlet to within the body cavity.

According to an aspect of the technology, there is disclosed, but not independently claimed, a method for delivering either one or more substances within at least one body cavity, characterized by steps of providing at least one pierceable vial with V_{sub} [ml or mg] of the substances; the vial having at least one fluid inlet port of diameter Dᵢₙ [mm] and at least one fluid discharging outlet port of diameter Dₒᵤₜ [mm], configured for placement in proximity to the body cavity; configuring the fluid inlet by means of size and shape to interface a puncturing member, so that upon coupling to the fluid inlet port, piercing of the same, thereby providing the substances in a fluid communication, with at least one chamber configured to accept pressurized fluid at volume V_{PF} [ml] and pressure P_{PF} [barg]; and facilitating the flow of the pressurized fluid from the chamber, via the fluid inlet, entrains the substances, erupts via the fluid discharging outlet port into the body cavity, such that the release time of the V_{sub} [ml or mg] of the substances and the V_{PF} [ml] of the pressurized fluid, dTᵣₑₗₑₐₛₑ is less than 500 milliseconds. The method further held the followings: V_{PF} is in a range of 1 to 50 ml; V_{sub} is in a range of about 0.01 to about 7 ml or 0.1 mg to 1 g; P_{PF} is in a range of about 0 to about 10 barg/ The following is further being held true: Dᵢₙ or Dₒᵤₜ are in a range of 0.2 to 6 mm; the pressure velocity is greater than 0.001 barg /ms; the pressure velocity is greater than 0.01 barg /ms; the volume rate or dV_{sub}/dTᵣₑₗₑₐₛₑ is greater than 0.0001 ml /ms; the volume rate dV_{sub}/dTᵣₑₗₑₐₛₑ is greater than 0.001 ml /ms; the volume rate dV_{PF}/dTᵣₑₗₑₐₛₑ is greater than 0.001 ml /ms; the volume rate dV_{PF}/dTᵣₑₗₑₐₛₑ is greater than 0.01 ml /ms; and any combination thereof. Additionally, the step of providing the vial additionally comprising step of selecting the vial from a group consisting of a pierceable container, a blow-fill-seal and a form-fill-seal and any combination thereof.

According to an aspect of the technology, said method additionally comprises at least one of the following steps: selecting the body cavity from a group consisting of a nasal cavity, the mouth, the throat, an ear, the eye, the vagina, the rectum, the urethra, and any combination thereof; selecting the gas from a group consisting of: air, nitrogen, oxygen, carbon dioxide, helium, neon, xenon, nitric oxide and any combination thereof; dispensing the at least one substance, and during the step of dispensing, forming a plume of aerosol with predetermined distribution from a mixture of the predetermined volume V_{gas} [ml] of the pressurized gas and the predetermined volume V_{sub} [ml] entrained within it; selecting the predetermined distribution from a group consisting of: a homogeneous distribution, a heterogeneous distribution; selecting the heterogeneous distribution from a group consisting of: an arbitrary distribution, a distribution in which the density of the at least one substance within the mixture follows a predetermined pattern, and any combination thereof; selecting characteristics of the aerosol from a group consisting of: particle size, particle shape, particle distribution, and any combination thereof, are determinable from characteristics of the device selected from a group consisting of: the predetermined volume of the pressurized gas, the predetermined volume of the substance, the predetermined pressure of the pressurized gas, the predetermined orifice size, and any combination thereof; selecting the substance from a group consisting of: a gas, a liquid, a powder, a slurry, a gel, a suspension, and any combination thereof; storing at least one the substance under either an inert atmosphere or under vacuum, thereby preventing reactions during storage; and characterizing a dose-response curve for brain concentration of the substance to be of substantially linear form; and a dose-response curve for brain concentration having a fit selected from a group consisting of logarithmic, parabolic, exponential, sigmoid, power-low, and any combination thereof; of the substance when administered nasally via the device.

### BRIEF DESCRIPTION OF THE DRAWINGS

The invention is herein described, by way of example only, with reference to the accompanying drawings, wherein:
**Figs. 1A-B** shows an embodiment of the present technology, with **Fig. 1A** showing an exploded view of the device and **Fig. 1B** showing the device fully assembled;
**Figs. 2A-C** shows another embodiment of the present technology, with **Fig. 2A** showing the device, **Fig. 2B** showing a cross section of the device and **Fig. 2C** showing an enlarged view of a portion of the device;
**Figs. 3A-D** shows another embodiment of the present technology, with **Fig. 3A** showing a cross-section of the device, **Fig. 3B** showing an enlarged view a portion of the device, **Fig. 3C** showing the exterior of the nosepiece and **Fig. 3D** showing the device from the top;
**Figs. 4A-C** shows another embodiment of the present technology, after activation, with the device shown in **Fig. 4A****,** a cross section of the device in **Fig. 4B** and an enlarged view a portion of the device in **Fig. 4C****;**
**Figs. 5A-G** show another embodiment of the device, with **Fig. 5A** showing a side view of a pre-used device carrying a BFS, **Fig. 5B** showing a cross section of the same, **Fig. 5C** and **5D** depicting the device when connected to a BFS, **Fig. 5E** showing the same when the device is ready to use, **Fig. 5F** showing connection of the BFS to the device; and **Fig. 5G** showing the device after activation;
**Figs. 6A-E** show another embodiment of the present technology, with **Fig. 6A** illustrating a front view of a pre-used device carrying a BFS and cross sections of the same; **Fig. 6B** showing securing of the BFS to the device and breaking the cap; **Fig. 6C** presenting a cross-section view; **Fig. 6D** showing a button at the base of the device being pushed; **Fig 6E** showing pressurized fluid flowing from the container to the BFS and carrying the drug outward;
**Figs. 7A-7B** illustrating a device being another embodiment of the present technology, a front and exploded view, respectively;
**Figs. 8A-8E** show same device in various modes of operation; and
**Fig. 9** depicts two views of the device interconnected to a safety latch according to an embodiment of the present technology.
**Figs. 10-19** show another embodiment of the present technology.
**Figs. 20-24D** show another embodiment of the present technology.
**Figs. 25-30D** show another embodiment of the present technology.
**Figs. 31-32C** show another embodiment of the present technology.
**Figs. 33-34D** show another embodiment of the present technology.
**Figs. 35A-35D** illustrating different embodiments of the hollow needle.
**Fig. 36A-36B** illustrating another embodiment of the present technology.

### DETAILED DESCRIPTION OF THE PREFERRED EMBODIMENTS

The following description is provided, alongside all chapters of the present invention, so as to enable any person skilled in the art to make use of the invention and sets forth the best modes contemplated by the inventor of carrying out this invention. Various modifications, however, will remain apparent to those skilled in the art, since the generic principles of the present invention have been defined specifically to provide a device capable of improving the transfer of medicament to a predetermined desired location and to provide a device capable of improving the delivery of medicament through the tissue.

In the present invention, a combination of parameters and forces such as pressure, gas/air volume orifice diameter enable the formation of optimized aerosol characteristics for both improved delivery of aerosol to the target area (such as the olfactory epithelium in the nasal cavity) and enhanced absorption at that area for better delivery to a desired tissue (such as the brain).

The term **'high barrier films'** hereinafter refers to any materials in flexible packaging laminations that prevent the permeation of water, water vapor, oil, oxygen, aroma, flavor, gas, or light. Such provision is enabled by low permeability of the film. In some embodiments the film is made of high-density Aluminum films. Thus, according to one embodiment of the present technology, the pressurized gas enclosed within high barrier film that can maintain the pressure of the compressed gas for prolong period of times without leakage therefrom.

The term '**ul**' or **'µm'** hereinafter refers to the unit micro liters or micro meters, respectively.

The term **'capsule'** interchangeably **'container'** interchangeably refer to a container configured to contain a flowable substance. The term flowable refers hereinafter to any liquid, gas, aerosol, powder and any combination thereof. It should be emphasized that the term capsule can also refer to a predefined volume within the same in which a flowable substance is placed. In other words, the predefined volume is sized and shaped to enclose a predefined volume of the substance.

The term **'plurality'** hereinafter refers to an integer greater than or equal to one.

The term **"olfactory epithelium'** hereinafter refers to a specialized epithelial tissue inside the nasal cavity. The olfactory epithelium lies in the upper top portion of the nasal cavity.

The term **'substance'** hereinafter refers to any substance capable of flowing. Such a substance can be a granular material, including a powder; a liquid; a gel; a slurry; a suspension; and any combination thereof. The term further refers to one or more members of a group consisting of proteins; stem-cells; cells, organs, portions, extracts, and isolations thereof; macro-molecules; RNA or other genes and proteins-encoding materials; neurotransmitters; receptor antagonists; biologic response modifiers; hormones; Ketamine; commercially available by Lilly (US) Baqsimi product; Glucagon, biologic response modifiers; Glucagon; substrates to treat one of eth followings: anaphylaxis, Parkinson, seizures and opioid overdose; epinephrine; atropine; metoclopramide; commercially available Naloxone or Narcan products; Esketamine (Spravato); Radicava [edaravone]; Ingrezza [valbenazine]; Austedo [deutetrabenazine]; Ocrevus [ocrelizumab]; Xadago [safinamide]; Spinraza [nusinersen]; Zinbryta [daclizumab]; Nuplazid [pimavanserin]; Aristada [aripiprazole lauroxil]; Vraylar [cariprazine]; Rexulti [brexpiprazole]; Aptiom [eslicarbazepine acetate]; Vizamyl [flutemetamol F18 injection]; Brintellix [vortioxetine]; Tecfidera [dimethyl fumarate]; Dotarem [gadoterate meglumine]; Antibody mediated brain targeting drug delivery including aducanumab, gantenerumab, bapineuzumab, solanezumab, ofatumumab CD20, BIIB033, LCN2, HMGB1; insulin; oxytocin; orexin-A; leptin; benzodiazepine i.e. midazolam; naloxone; perillyl alcohol; camptothecin; phytochemicals including curcumin and chrysin; nucleotides; olanzapine; risperidone; Venlafaxin; GDF-5; zonisamide; ropinirole; plant-originated and synthetically-produced terpenes and cannabinoids, including THC and CBD; valproric acid; rivastigmine; estradiol; topiramate or an equivalent preparation comprising CAS No. 97240-79-4; MFSD2 or MFSD2A or sodium-dependent lysophosphatidylcholine symporter; and any esters, salts, derivatives, mixtures, combinations thereof, with or without a carrier, liposomes, lyophilic or water-miscible solvents, surfactants, cells, cells fractions, at a therapeutically effective concentration.

The term **'gas'** refers to any fluid that can be readily compressed. Gases as used herein include, but are not limited to, air, nitrogen, oxygen, carbon dioxide, helium, neon, xenon, nitric oxide and any combination thereof. Devices charged by hand will typically use air as the carrier gas.

The term **'fluid'** refers to any substance or mixtures of substances that continually deforms (flows) under an applied shear stress, or external force. This term refers to gas, liquids, particulate or granulated solids (powders), aerosols, and any mixtures and combinations thereof.

The term **'about'** refers hereinafter to a range of 25% below or above the referred value.

The term **"body orifice"** and **"body cavity"** are interchangeably refer to one or more of the followings: nasal cavity, a mouth, a throat, an ear, the eye, a vagina, a rectum, a urethra, and any combination thereof.

The term **'biologic'** or **'biologic response modifier'** hereinafter refers to material manufactured in or extracted from biological sources such as a genetically engineered protein derived from human genes, or a biologically effective combination of such proteins.

All pressures herein are gauge pressures, relative to atmospheric pressure. Pressure units will be written herein using the standard abbreviation for "gauge', namely, "g". For example, atmospheric pressure is 0 barg and a pressure of 1 bar above atmospheric is 1 barg.

The term **'release time'** refers hereinafter to the time for the drug and carrier gas to substantially completely exit the device. Typically, the release time is affected by the combination of the Volume of substance, volume of pressurized gas, pressure of pressurized gas, the orifice diameter, the activation time of the valve that reflects the time for the device to reconfigure from the ACTIVE configuration to the INACTIVE configuration or vice versa and any combination thereof.

The terms **'the device', 'the present device', 'the SipNose device'** and **'SipNose'** will be used interchangeably to refer to a device according to any embodiment of the present technology.

In all of the embodiments of the device shown hereinbelow, identical numbers refer to identical functions. All figures shown herein are illustrative and none is to scale.

The present disclosure teaches a device for delivering a predetermined amount of a substance, preferably comprising a medication or combination of medications, into a body orifice of a subject, the orifice comprising any of the body's natural orifices, including a nostril, the mouth, the ear, the throat, the urethra, the eye, the vagina, the rectum and any combination thereof.

In preferred embodiments of the device, the device comprises a delivery mechanism and a medicament capsule, as described hereinbelow. The device can apply a broad range of drugs and materials to the nasal cavity for local effect, deliver a broad range of drugs and materials through the nasal cavity to the systemic circulation, deliver a broad range of drugs and materials through the nasal cavity to the central nerve system (CNS) the brain, spinal cord and associated nerves, and any combination thereof.

The drugs to be applied could be, but are not limited to, pharmaceuticals, natural compounds, biologics, hormones, peptides, proteins, viruses, cells, stem cells and any combination thereof.

However, it should be emphasized that the device can be provided alone as well as in combination with a capsule.

In some cases, the capsule would be provided with a known medicament within the same and in other cases the capsule would be 'filled' with the medicament just before use.

In some embodiments of the present technology, the device operating characteristics and the substance characteristics can be jointly optimized to maximize uptake of the substance at the desired site. In preferred variants of such embodiments, uptake is further optimized by exploiting synergies between delivery characteristics generated by the device and by the formulation or composition of the delivered material

In some embodiments, the substance comprises one or more agents to optimize delivery through the mucosal membrane by means of mucoadhesive agent and/or a permeability enhancer agent and/or a particulate formulation in the nano-particle or macro-particle range, and any combination thereof. In such embodiments, the combination of the device and substance enhance the delivery of the active agent to the target area (nasal epithelium and more specifically olfactory epithelium) and from there to the target tissue (for example the brain).

A non-limiting example is a composition comprising a drug to be delivered and at least one chemical permeation enhancer (CPE). In a preferred embodiment, the composition contains two or more CPEs which, by using a nasal delivery device, affect in an additive manner or behave synergistically to increase the permeability of the epithelium, while providing an acceptably low level of cytotoxicity to the cells. The concentration of the one or more CPEs is selected to provide the greatest amount of overall potential (OP). Additionally, the CPEs are selected based on the treatment. CPEs that behave primarily by transcellular transport are preferred for delivering drugs into epithelial cells. CPEs that behave primarily by paracellular transport are preferred for delivering drugs through epithelial cells. Also provided herein are mucoadhesive agents that enable the extension of the exposure period of the target tissue/ mucus membrane to the active agent, for the enhancement of delivery of the active agent to and through the mucus membrane.

In contrast to prior-art nasal delivery devices and technologies, the devices of the present technology can produce a fine aerosol in the nasal cavity or other desired body orifice at the target area and at the location of the target tissue instead of producing the aerosol only within the device or immediately after exit from the device. Utilizing the pressure as a driving force and the air as a carrier allows the material to be released from the nozzle as a mixture of aerosol and a pre-aerosolized state. The properties of the resultant aerosol are typically dependent on the properties of the device and of the medium into which the device is discharged. The properties of the device which affect the aerosol characteristics are the delivery pressure, the volume of the delivery gas, the characteristics of its orifice and time of activate.

In some embodiments, the aerosol properties are fairly independent of the delivered substance, while, in other embodiments, the pressure, volume, orifice characteristics, and delivered substance properties can be co-optimized.

In prior-art devices the aerosol is produced in proximity exit of the device. Typically, the aerosol comprises a wide "fan" of aerosol and a low driving force. Therefore, large droplets typically deposit very close to the exit from the device, while smaller droplets tend to quickly contact the walls of the passage, so that deposition is typically predominantly close to the delivery end of the device, with little of the substance reaching desired sites deeper in the body orifice, such as the middle and superior turbinates of the nose. In the present technology the aerosol created, due to the pressurized air carrier, reaches the upper regions of the nasal cavity.

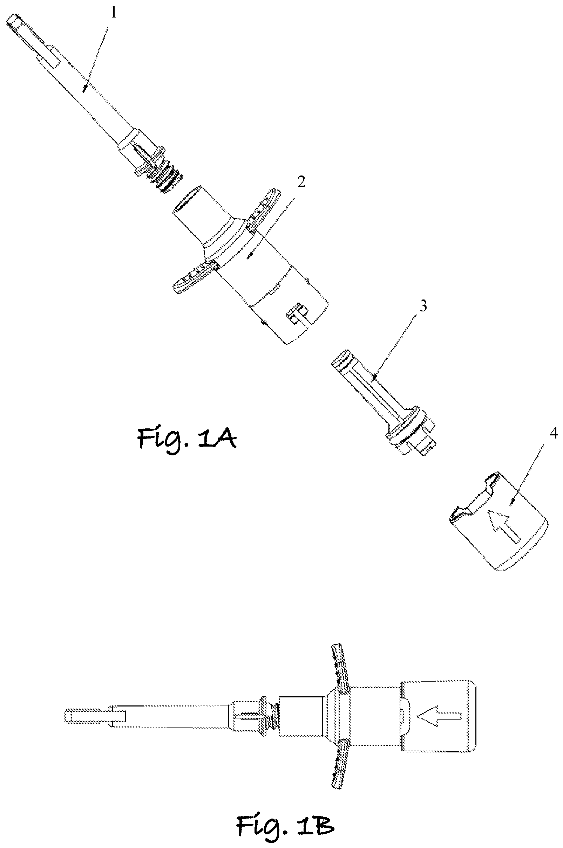

Reference is now made to **Fig. 1A-B,** disclosing a device according to one embodiment of the present technology. **Fig. 1A** shows an exploded view of the device, while **Fig. 1B** shows the device fully assembled. As shown in **Fig. 1A****,** the device comprises, inter alia, a BFS nose piece **(1),** a pressurized-fluid container **(2),** an air chamber gate **(3)** and an activation mechanism base **(4).**

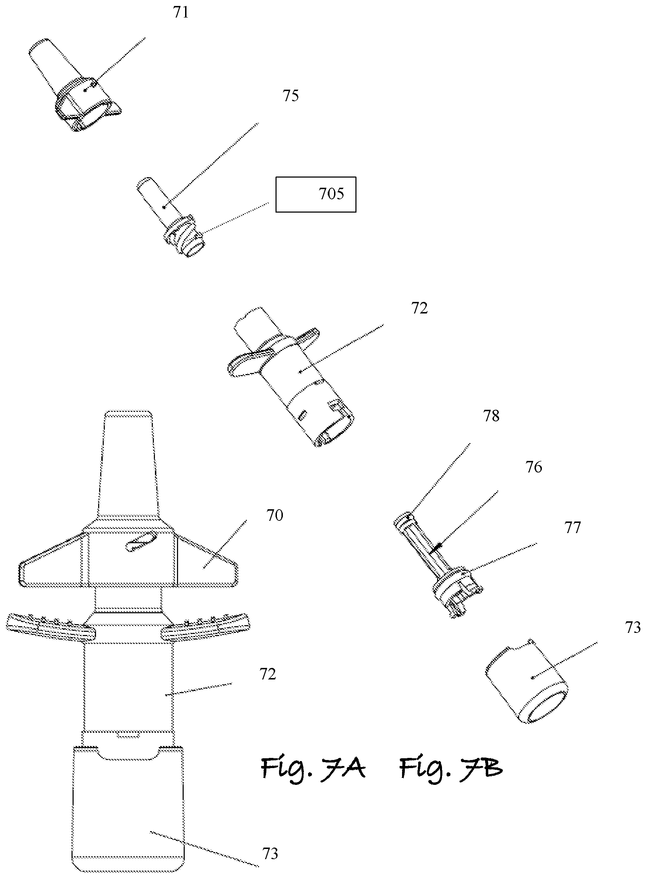

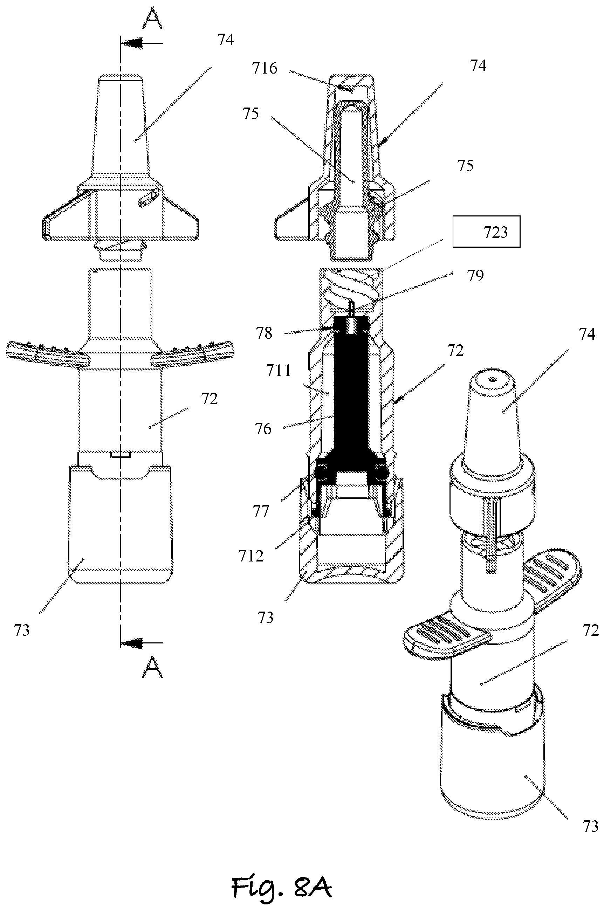

In **Fig. 1A****,** the base **(4),** an air chamber gate **(3)** has with a first gate O-ring at its proximal end and a second gate O-ring at its distal end (both shown in **Figs. 7A-7B** and **8A-8E,** as numerical reference **77** and **78).**

The pressurized-fluid container **(2)** will fit over the air chamber gate **(3),** with the first gate O-ring and the second gate O-ring providing airtight seals before activation so that compressed gas is storable between the air chamber gate **(3)** and the pressurized-fluid container **(2).**

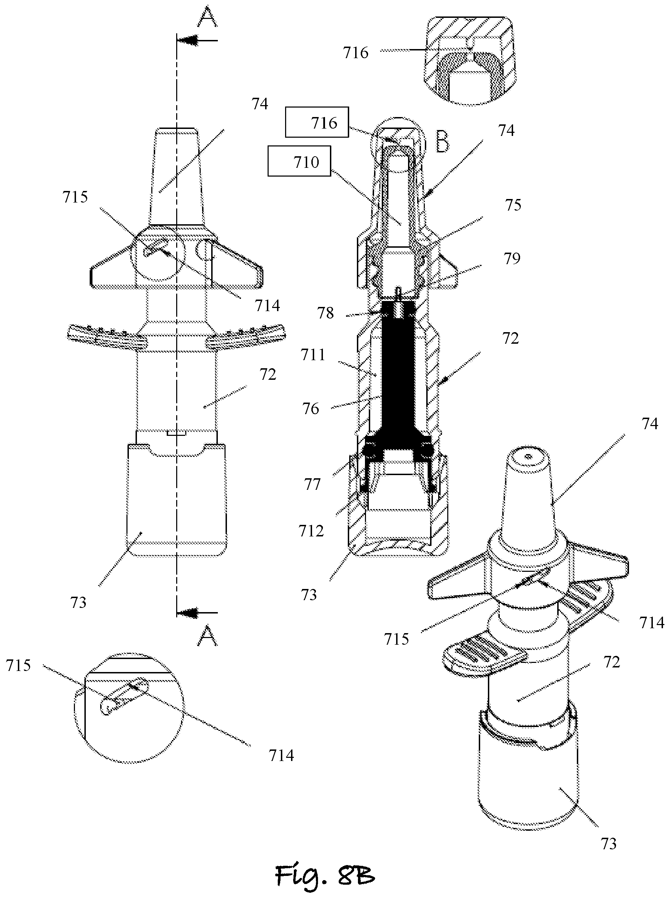

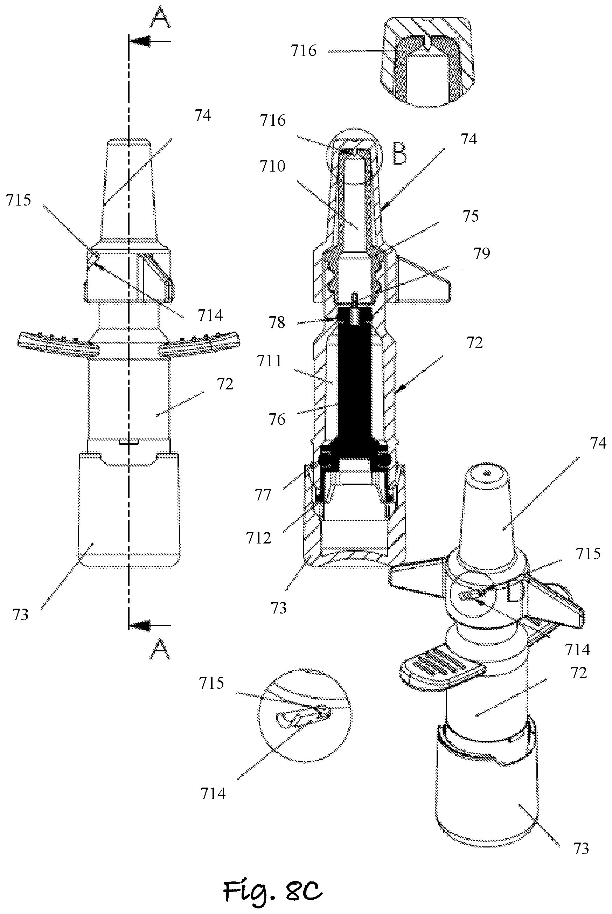

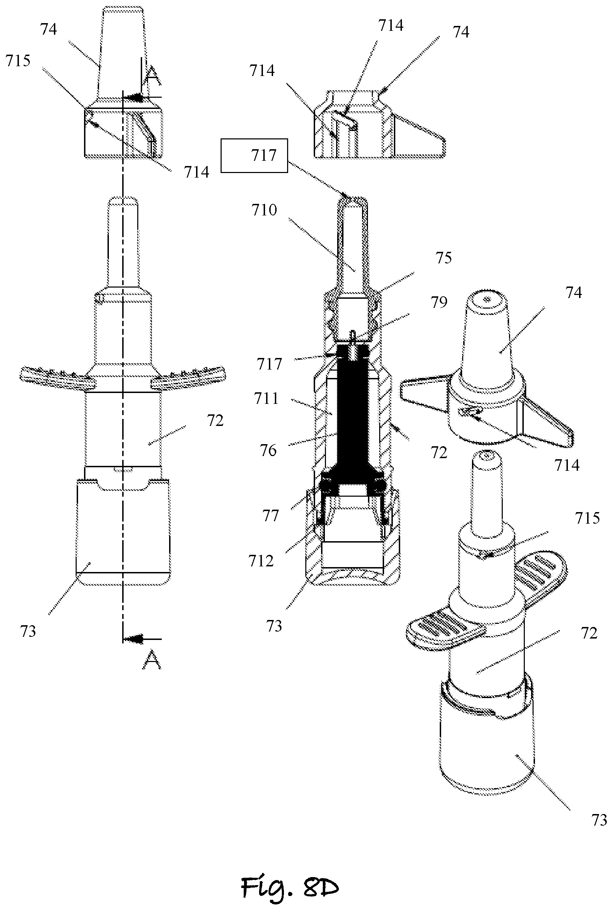

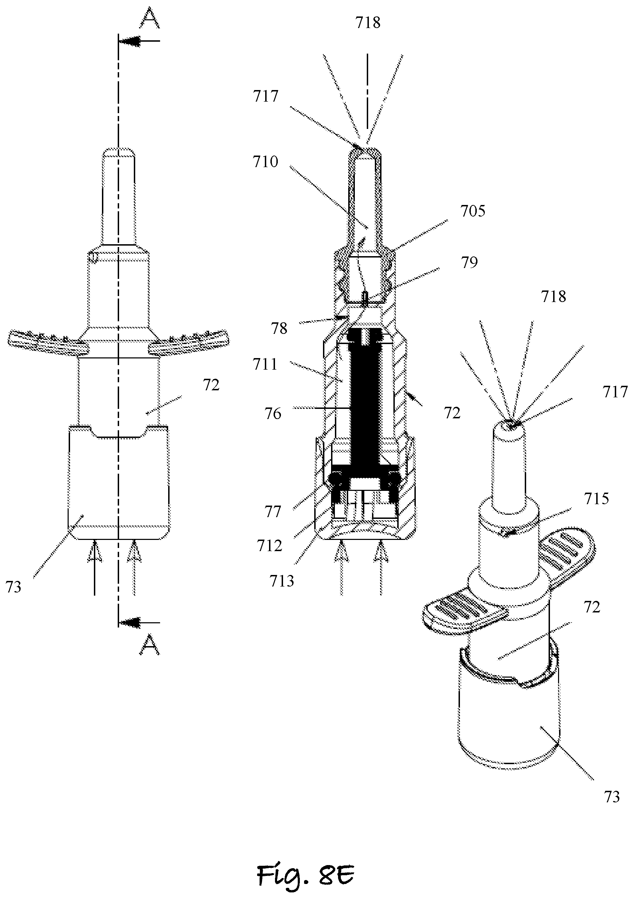

As will be disclosed hereinafter, the pierceable drug container (**1**) (e.g., BFS ) in the nosepiece, where there is a puncturing element that punctures the drug container and once the compressed gas is released from the pressurized-fluid container **(2),** the same entrains the drug and deliver the same to the nasal cavity (see **Figs. 7A-7B** and **8A, 8B, 8C, 8D** and **8E).**

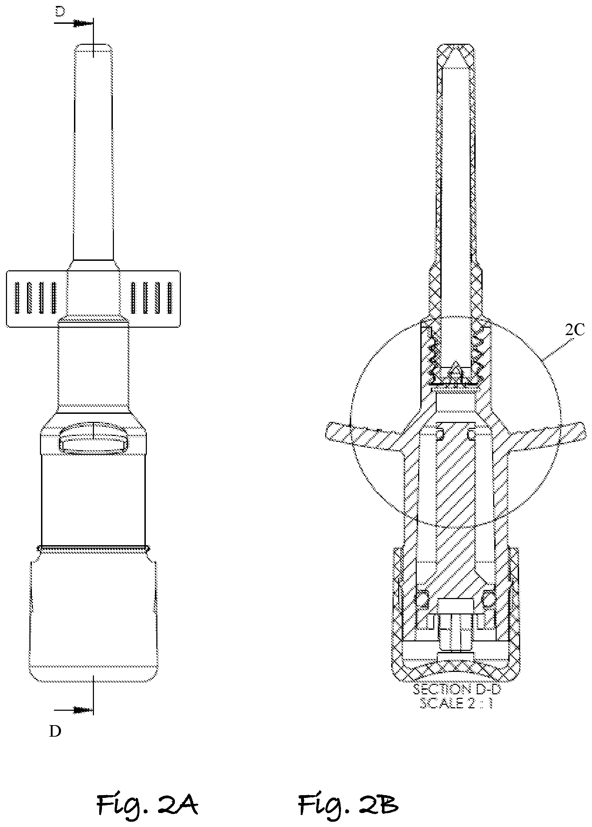

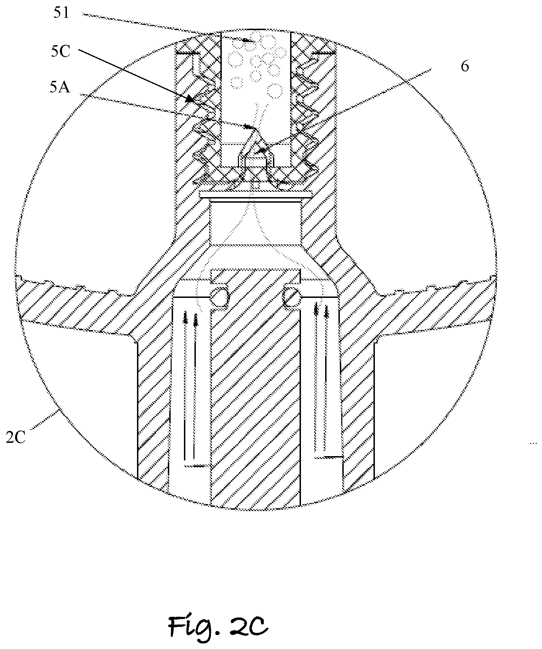

As shown in the drawings, the base of the device forms the activation button **(4);** to activate, the activation button **(4)** is pressed upward, then the air chamber gate **(3)** is drawn downwardly, which removes the sealing of the upper O-ring (78 in **Figs. 7A-7B****).** The movement of the air chamber gate **(3)** opens a gap between the pressurized-fluid container **(2)** and the BFF nose piece **(1),** allowing the pressurized-fluid to escape from container 2, enter BFF nose piece **(1)** (after the same has been pierced by the piercing needle 79, shown in **Figs. 7A-7B****),** and entrain the substance to the nasal cavity. Reference is now made to **Figs. 2A-C,** disclosing a device according to another embodiment of the present invention. **Fig. 2B** depicts a cross section along the line **D:D** of the device as shown in **Fig. 2A****.** The area within the circle **2C** in **Fig. 2B** is shown enlarged in **Fig. 2C****,** where the device's spike is disclosed **(6).** Also seen in **Fig. 2C** is a BSF lower BFS point at which the needle punctures the BFS**(5A),** BSF nosepiece which contain the drug **(51)** and an activation screw mechanism **(5C).**

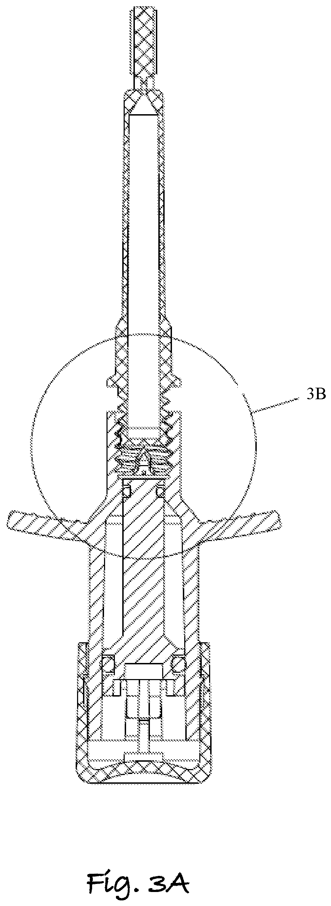

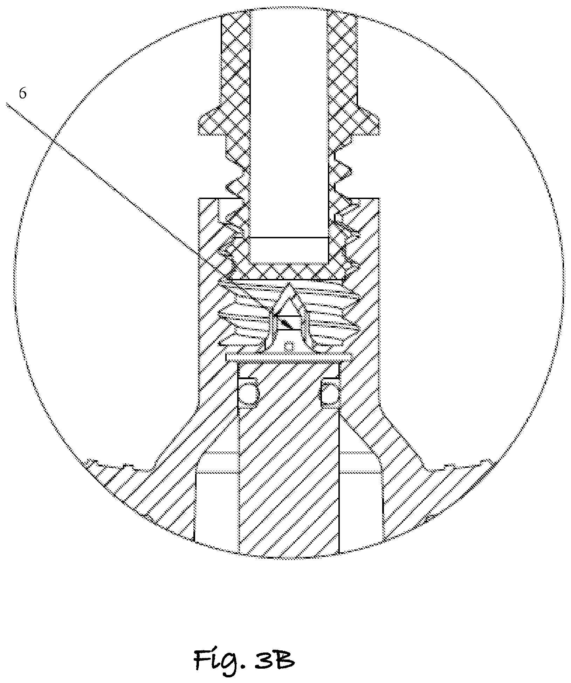

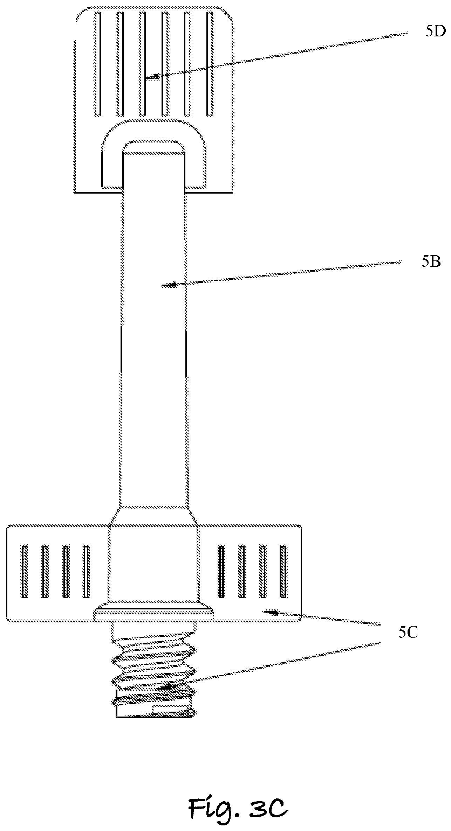

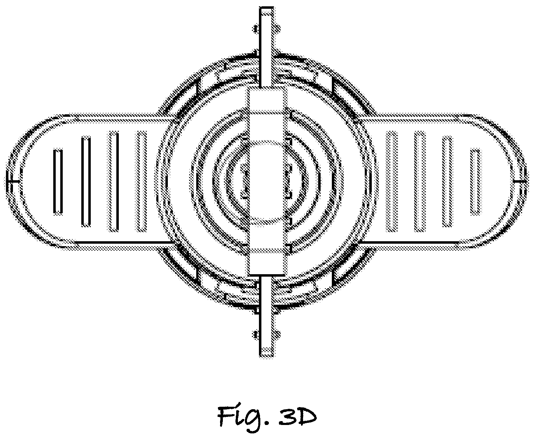

Reference is now made to **Figs. 3A-D,** disclosing a device according to another embodiment of the present technology. **Fig. 3A** shows a cross-section of the device. **Fig. 3B** shows an enlarged view of the area inside the circle **3B** of **Fig. 3A****.** The piercing member **(6)** can be clearly seen. **Fig. 3C** shows the exterior of the nosepiece, showing the activation screw mechanism **(5C)** that is tightened in order to drive the bottom of the drug container against the spike and thereby pierce the drug container; the nosepiece cover **(5D)** and the main body of the nosepiece **(5B).** **Fig. 3D** shows the device from the top.

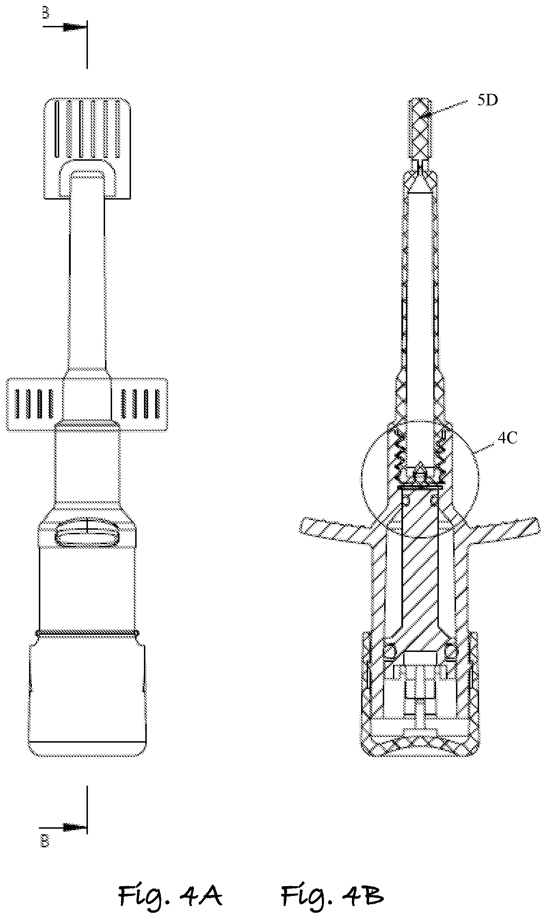

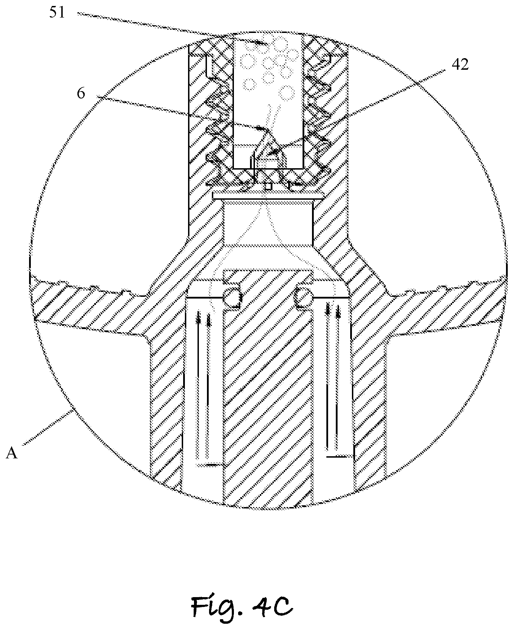

Reference is now made to **Figs. 4A-C,** disclosing a device according to another embodiment of the present technology. Here a device is after the activation **(****Fig. 4A). Fig. 4B** shows a cross section of the same along the line **B:B.** The area within circle **4C** is shown enlarged in **Fig. 4C****,** namely a cross section of the piercing member. Drug powder and/or liquid schematically illustrated **(51).** Air flow through the spike holes **(42)** entrains the drug.

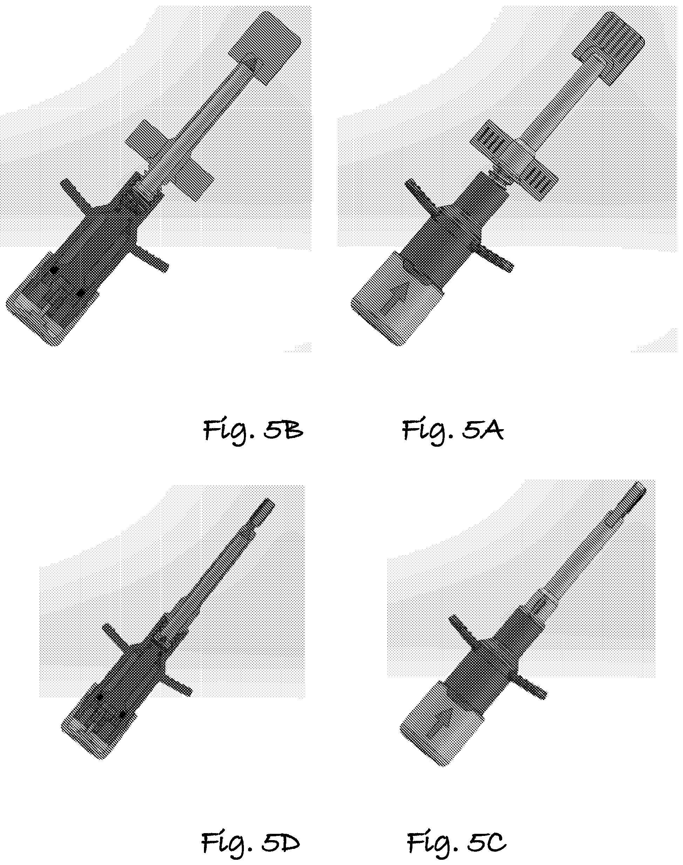

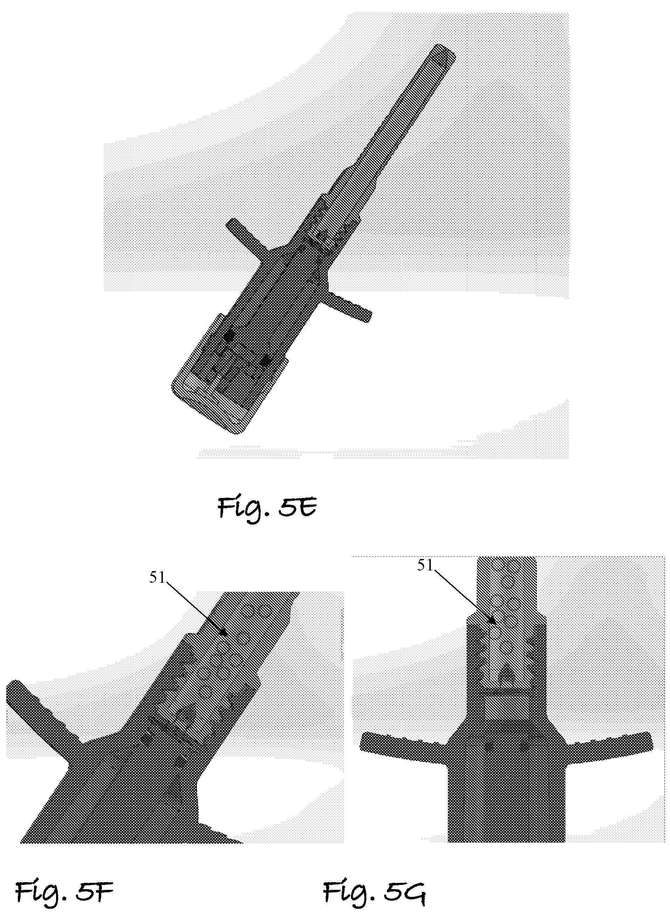

Reference is now made to **Figs. 5A-G,** disclosing a device according to another embodiment of the present technology. **Fig. 5A** is a side view of a pre-used device carrying a BFS, and **Fig. 5B** is a cross section of the same. **Fig. 5C** and **5D** similarly depict the device when connected to a BFS. **Fig. 5E** shows the same when the device is ready to use, **Fig. 5F** illustrates the connection between the BFS to the device; drug **(51)** is shown. The device after activation presents the flowing drug **(51)** in **Fig. 5G****.**

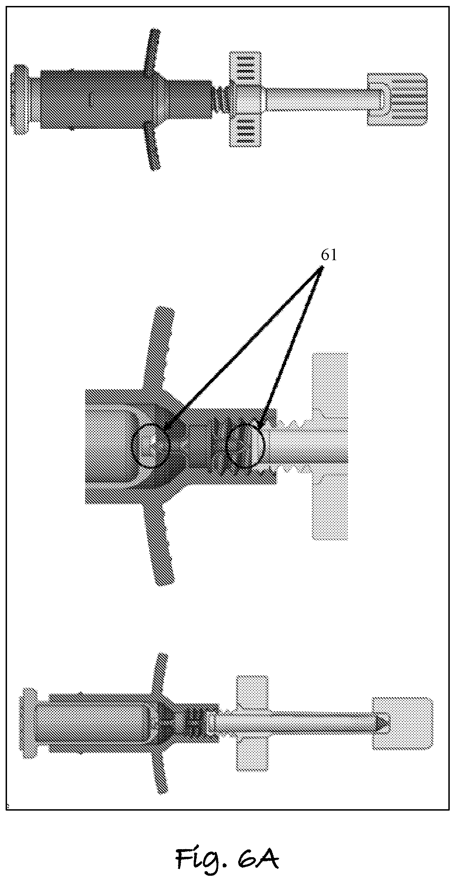

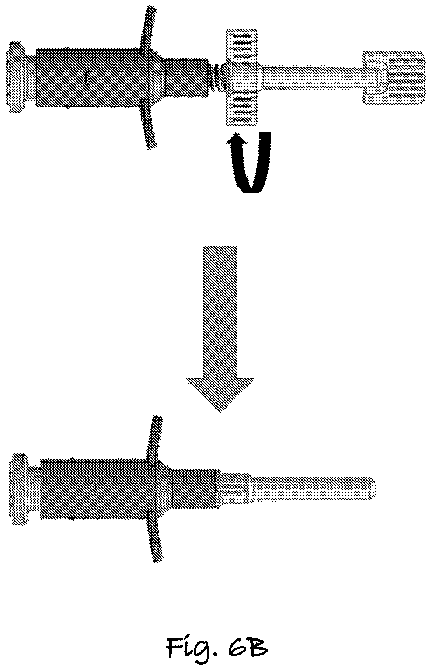

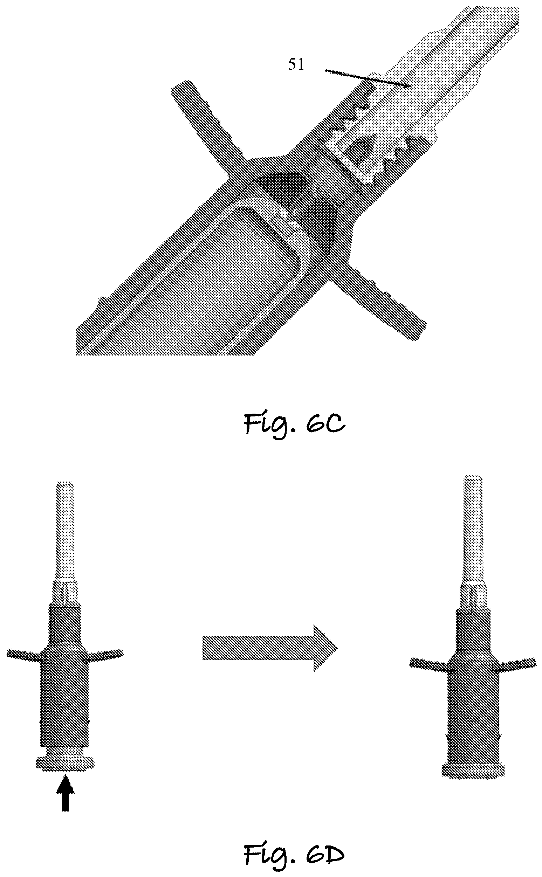

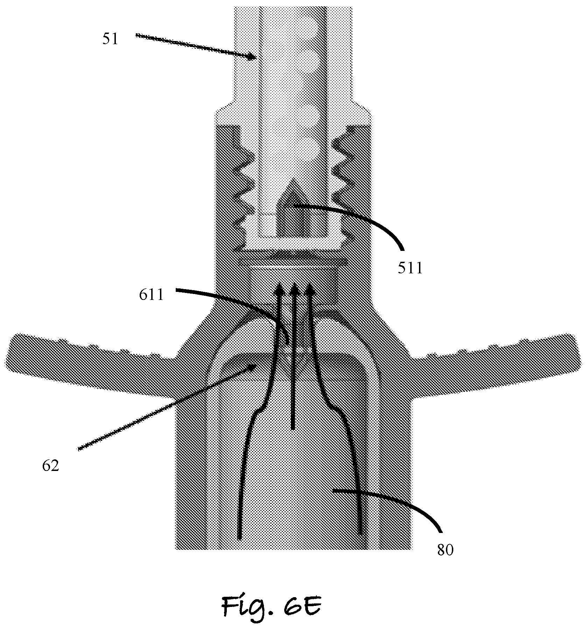

Reference is now made to **Figs. 6A-E** disclosing a device according to another embodiment of the present technology. **Fig. 6A** illustrates a side view (image on the top) of a pre-used device carrying a BFS. Images on the middle and in the bottom are cross sections of the same, showing BFS nosepiece and BFS air container before contact. **Fig. 6B** shows the second step after introducing the BFS, namely securing the BFS to the device, here by turning the nosepiece of the BFS clockwise. Upon rotation of the nosepiece, the piercing member **511** (shown in **Fig. 6C****),** pierces the drug compartment. A further step is removing (e.g., breaking) the cap, the image at the bottom presents the device after breaking the said cap. The drug **(51)** is presented in cross section view of **Fig. 6C****.** In **Fig. 6D****,** a button at the base of the device is pushed. Such push actuates the base and a second piercing member **611** pierces the container 80. Subsequently, as depicted in **Fig. 6E****,** pressurized fluid (air, nitrogen etc.) flows from its container **(62)** to the drug-containing BFS and carries the drug (liquid phase, solid powder particles etc.) **(51)** outwardly.

One should also note that this example is shown for the same invention but with another kind of pressurized gas container and a different way of compressed gas discharge (by puncturing the container rather than the gate that is shown in the previous figures.

Reference is now made to **Fig. 7A, Fig. 7B** and **Figs. 8A-8E** disclosing a device according to another embodiment of the present technology in a side view and exploded view, respectfully; wherein **70** is a cover holding area; **72** is a pressurized fluid container; **73** is an activation mechanism base; **74** is a cover's body; **75** is a nosepiece; **76** is an air chamber gate; **78** and **77** are O-rings; **79** is a needle; **75** is a nosepiece one way screwing mechanism; **710** is a drug's space; **712** is an air chamber gate's legs; **713** is an air chamber gate's snaps; **714** is a drug storage container locking notch; **715** is a drug storage container locking pin; **716** is an orifice-creating piercing needle; **717** is an orifice; **718** is an aerosol; **720** is a safety latch; **721** and **722** locks; **723** is a pressurized fluid container's internal screwing mechanism. The device comprises modules **70-79** and **710-720,** where **71, 74,** and **713,** and nozzle (orifice) **717** are related with the nosepiece; **72, 76-79, 710-712, 716,** to the body and module **73** and **713** is in device's operating button.

In the following sets of figures, namely **Fig. 8A-8E****,** operation modes are illustrated, illustrating a method for delivering either one or more substances within at least one body cavity, characterized by steps of providing a vial with V_{sub} [ml] of said substances; said vial selected from a pierceable container, a blow-fill-seal and a form-fill-seal, further providing said vial with a fluid inlet and a fluid discharging outlet of diameter D [mm], configured for placement in proximity to said body cavity; configuring said fluid inlet by means of size and shape to interface a puncturing member, so that upon coupling to the fluid inlet, piercing of the same, thereby providing the substances in a fluid communication, via a valve, with a chamber configured to accept pressurized fluid at volume V_{PF} [ml] (or mg) and pressure P_{PF} [barg]; the valve is commutable from an CLOSE to an OPEN CONFIGURATION within a short period of time, <500 milliseconds (dT); at the OPEN CONFIGURATION, facilitating the flow of the pressurized fluid to from the chamber, via the fluid inlet, entraining the substances; and then erupting via the fluid discharging outlet to within the body cavity, such that the release time of the V_{sub} [ml or mg] of the substances and the V_{PF} [ml] of the pressurized fluid, dTᵣₑₗₑₐₛₑ is less than 500 milliseconds. Each pf the figures comprises a front view (left side), cross-section **(A:A,** middle) and isometric view (right side). **Fig. 8B** and **8C** further depict a rotation mechanism **714-715,** which allows a rotation (here, ¼ rotation) thereby enabled the piercing of the nosepiece substance container.

According to another embodiment, the rotation results in a double piercing of the nosepiece substance container and the pressurized air container.

According to another embodiment the pressurized air container is sealed by means of at least one O-ring, such that movement of the o-ring removes the sealing and enables the release of the pressurized air. In some embodiment at least 2 o-rings are used. One o-ring at the bottom of the pressurized air container and the second at the upper portion of the pressurized air container to seal and separate between the pressurized air container and the nosepiece substance container.

At final step **(****Fig. 8E****),** upon pressing the activation mechanism base **73,** results in movement of the air chamber gate 76 and the upper o-ring to thereby enable the release of the pressurized air from the pressurized air container and into the nosepiece substance container to entrain the same. Once the pressurized air entrains the substance, aerosol **718** is provided throughout the orifice **717,** having a narrow plume angle (θ). It is in the scope of the technology wherein the cover comprises means to protect the drug from UV, e.g., photoprotective agents, such as oxybenzone, titanium oxide and octyl methoxycinnamate.

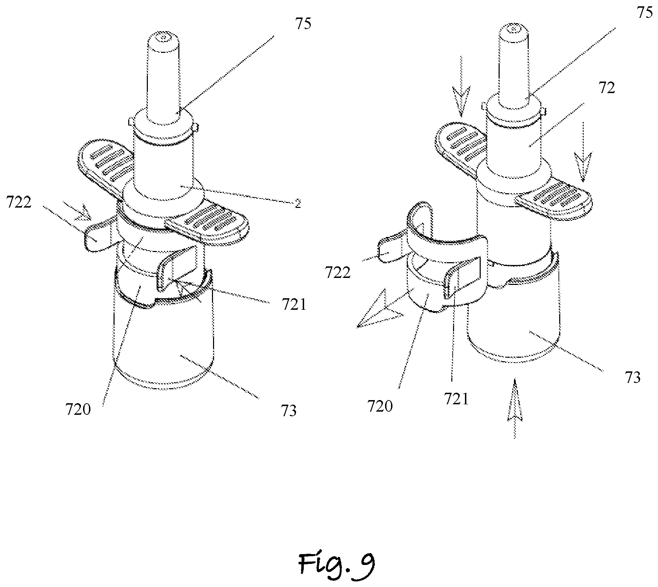

Reference is now made to **Fig. 9** disclosing a device according to another embodiment of the present technology, where the device comprises a safety latch **720** with its two locks **721-722,** configured to avoid undesired or accidental operation of the device, i.e., by pressing activation mechanism base **73** and pressurized fluid container (body) **72.**

It is well in the scope of the technology wherein the pressurized fluid is accommodated within container for a respectively long time, e.g., by having a pre-pressurized container **(step 1A)** in a fluid connection **(step 2A)** with the BFS and releasing the same **(step 3A),** or alternatively a container suitable to pressuring the fluid in situ within the container, e.g., by introducing a pump or piston mechanism that pressuring ambient air to the container in a first step **(step 1B)** and accommodating the pressurized fluid along a relatively short time of **step 2B,** then free the fluid to flow in **step 3B.**

By the device disclosed herein, the pre-aerosolized mixture of gas and substance exits the device with a significant driving force as a mixture of aerosol and pre-aerosolized material (fluid or powder). When the pre-aerosolized material hits the walls of the nasal passages, it "explodes" into a fine aerosol that is capable of being driven by the pressure deep into the nasal passages to deposit in the desired region.

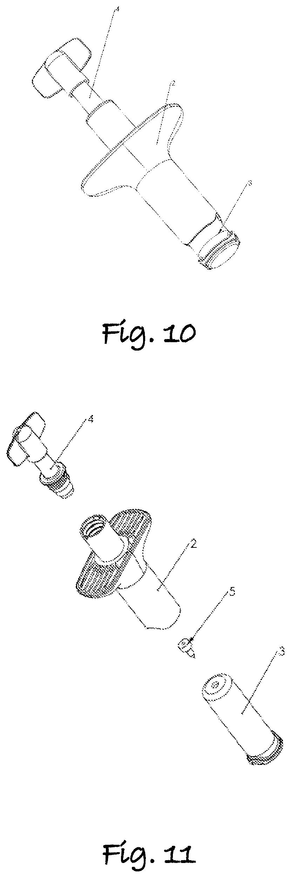

Reference is now made to **Figs. 10-11** which illustrates another embodiment of the present technology, in which the pierced container is the pressurized gas container.

**Fig. 10** illustrates the nosepiece 4 (containing the medicament container), the handle, the device body 2 and the pressurized air container 3.

Reference is now made to **Fig. 11****,** illustrating an embodiment in which the pressurized gas container 3 is pierced by means of a dedicated hollow needle 5.

According to this embodiment, once the device is activated (the pressurized gas container 3 is pressed against the handle), needle 5 pierces the pressurized gas container 3 and the pressurized gas exits therefrom and enters the medicament container to entrain the medicament into the nasal cavity.

Needle 5 is a hollow needle such that when the same pierces the gas container, the pressurized gas exit the pressurized gas container 3 throughout needle 5.

Needle 5 comprises two ends, one of which is a flat, non-sharp end, in fluid communication with the medicament container (will be disclosed hereinafter) and the second one has a sharp end, adapted to pierce the gas container. The two ends interconnected by means of a hollow tube. Once the gas container is pierced the pressurized gas exits therefrom through the hollow tube.

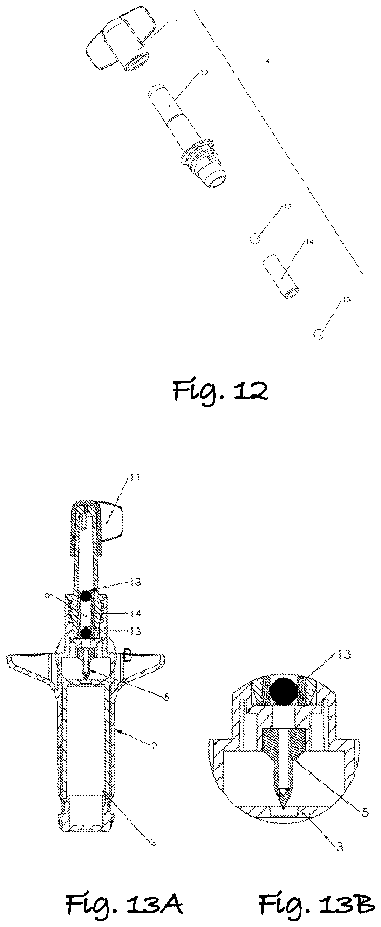

Reference is now made to **Fig. 12** illustrating one embodiment of the nosepiece 4 enclosing a powder medicament. According to this embodiment the nosepiece has a cap 11 adapted to cover the distal most part of the nosepiece body 12. Within the nosepiece body 12, a dedicated medicament capsule 14 is disposed. The medicament capsule 12 is adapted to enclose e.g., powder medicament. However, one skilled in the art would acknowledge that also liquid, gas or gel medicament are also within the scope of the present invention. Furthermore, capsule 12 can enclose a combination of both liquid and powder medicaments.

According to this embodiment, at least one spherical element, preferably ball 13, is disposed within the medicament capsule 12. According to another embodiment, at least two spherical elements, preferably ball 13, are disposed within the medicament capsule 12, such that the medicament are disposed between the two balls 13.

The spherical elements are adapted to both (a) seal the medicament capsule 12 and prevent leakage of medicament therefrom; and, (b) once the pressurized air exits the pressurized gas container, the bottom most special element 13 is removed from its position (thereby removing the sealing thereof and enabling the pressurized gas to enter therein) and mixes/ compress the medicament enclosed between the two spherical elements 13. Once, the first (bottom most) spherical element is removed, the same compress the medicament disposed between the first and second spherical elements 13 and cases the removal of the second (upper most) spherical element 13. Thereafter, the medicament along with the pressurized gas are delivered to the nasal cavity.

Reference is now made to **Figs. 13A-13D** illustrating the step-by-step activation of the device. **Fig. 13A** illustrates the device before activation, in which the medicament is enclosed within the medicament container 14 and the gas container is not pierced yet. **Fig. 13B** provides a closer view of the needle 5 before activation thereof.

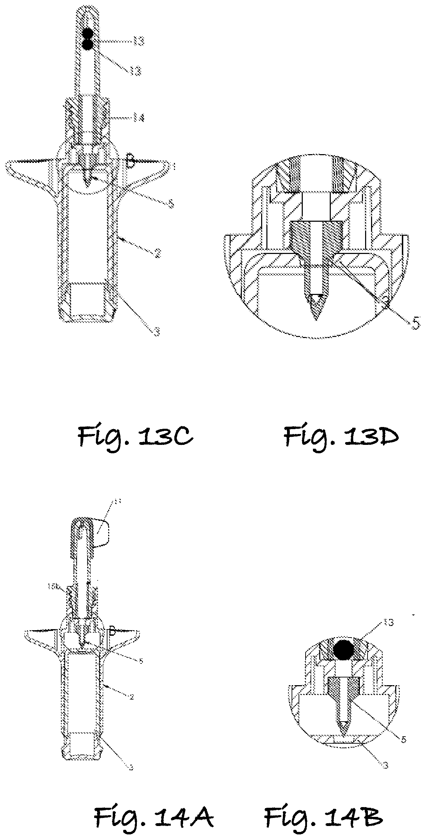

**Fig. 13C** illustrates the device after activation, in which the gas container is pierced, the pressurized gas had entered the medicament container, removed the two spherical elements 13 (the two spherical elements 13 are shown outside capsule 14) to enable delivery of the medicament 15. **Fig. 13D** provides a closer view of the needle 5 after activation thereof (i.e., pierced the gas container).

Reference is now made to **Figs. 14A-14B** illustrating other embodiment of the present technology in which liquid medicament are utilized. According to this embodiment, only one spherical element 13 is utilized. Said spherical element 13 is disposed in the bottom most part of the medicament capsule to seal the same. Once, the needle 5 pierces the gas container, the pressurized gas exits the gas container through the hollow needle to remove the spherical element, entrain the medicament and deliver the same to the nasal cavity. **Fig. 14A** illustrates the device before activation, in which the medicament is enclosed within the medicament container 14 and the gas container is not pierced yet. **Fig. 14B** provides a closer view of the needle 5 before activation thereof.

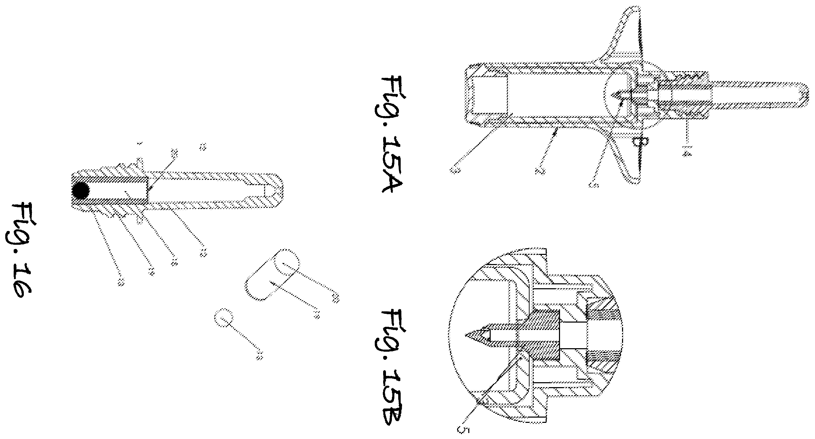

**Fig. 15A** illustrates the device after activation, in which the gas container is pierced, the pressurized gas had entered the medicament container, removed two spherical element 13 (the two spherical elements 13 are shown outside capsule 14) to enable delivery of the medicament 15. **Fig. 15B** provides a closer view of the needle 5 after activation thereof (i.e., pierced the gas container).

Reference is now made to **Fig. 16****,** which illustrates a closer view of one embodiment of a capsule enclosing either liquid medicament or powder or 15 and the spherical element 13. According to one embodiment a combination of liquid medicament and powder can be enabled by displacing e.g., the powder medicament on the bottom part of the capsule and the liquid medicament in the upper part thereof and vice versa.

According to this embodiment, the capsule comprises the spherical element 13 being displaced in the bottom most part of the capsule (providing sealing in the bottom part) and a breakable membrane 20 in the upper part thereof (providing sealing in the upper part).

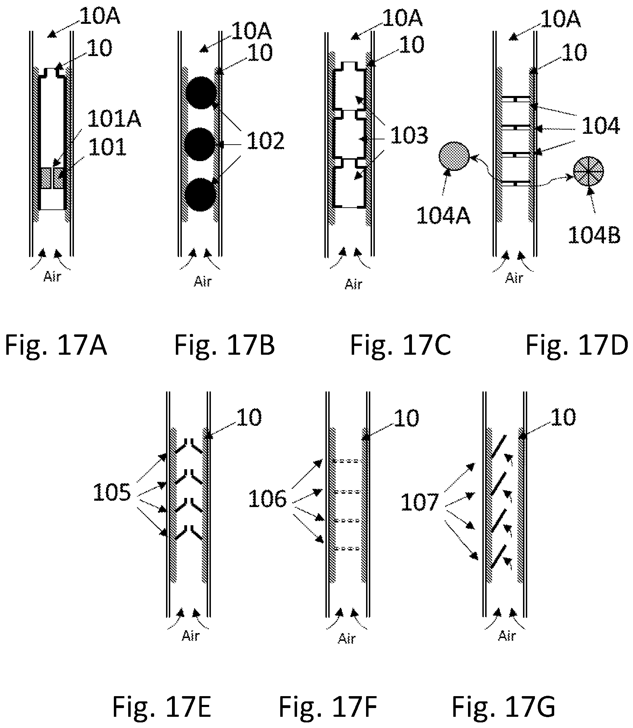

It should be noted that different embodiments of the capsule are available. Reference is now made to **Figs. 17A-17G****.**

**Fig. 17A** shows a plunger-type barrier **(101)** between compartments. In this exemplary embodiment, there is one plunger **(101).** In other embodiments, more or fewer plungers **(101)** can be present. The plunger **(101)** comprises a hole or slot small enough to prevent passage of substance therethrough, but wide enough to allow passage of compressed air therethrough. When the device is activated, compressed gas (curved arrows at bottom) enters the capsule **(10).** The pressure forces the plunger **(101)** upward, forcing substance above the plunger **(101)** out of the top of the capsule. Substance below the plunger **(101)** will be forced upward by the compressed air, to mix with the substance above the plunger in a nose piece (not shown). The plunger **(101)** passes through the top of the capsule into an intermediate space **(10A)** below the nosepiece (not shown; a shoulder or other barrier (not shown) prevents the plunger **(101)** from exiting the nosepiece.

The hole or slot **(101A)** in the plunger **(101)** is narrow enough to prevent substance leakage during storage, and wide enough to allow compressed gas passage during activation, wiping the substance from the container during activation. The hole or slot **(101A)** in the plunger **(101)** can be designed in many ways to allow delivery that is very efficient, having a residual volume of less than 15% of the original volume. The plunger **(101)** can be made either from a flexible materials such as, but not limited to, silicone, rubber, flexible plastic or from a hard material such as, but not limited to, a polymer such as Delrin^{®}, a plastic, nylon, metal and any combination thereof.

**Fig. 17B** shows ball-type barriers **(102)** between compartments. The balls **(102)** provide both a separation function, before activation, and a mixing function during activation. In this exemplary embodiment, there are 3 balls **(103).** In other embodiments, more or fewer balls **(103)** can be present. When the device is activated, compressed gas (curved arrows at bottom) enters the capsule **(10).** The pressure forces the balls **(102)** upward, forcing substance above the topmost ball **(102)** out of the top of the capsule. The topmost ball **(102)** passes through the top of the capsule into an intermediate space **(10A)** below the nosepiece (not shown; a shoulder or other barrier (not shown) prevents the balls **(102)** from exiting the nosepiece. The substance between the first and second balls can then pass through the top of the capsule **(10)** into the nosepiece (not shown, and mix with the first substance. The second ball **(102)** can then enter the intermediate space **(10A),** and similarly with all balls **(102)** in the capsule **(10)** until the capsule **(10)** is empty.

Ball-type barriers **(102)** are useful when mixing of several components should occur only upon delivery, when one or more substance should be maintained at low humidity, when the viscosity of the substance varies significantly, and any combination thereof. In addition, contact between the ball **(102)** and the walls of the capsule **(10)** can also ensure effective release of the substance from the capsule **(10).** Examples of substances which tend to cling to walls include, but are not limited to, oils and some powders. The barriers can be balls, as in the embodiment shown, angular dividers or any other shape which can be easily moved by the released compressed gas (low-friction contacts), and still provide effective sealing between the elements to avoid mixing during, for example, shipment and storage.

**Fig. 17C** shows an embodiment with linked drug containers **(103)** within the capsule **(10).** In this exemplary embodiment, there are 3 linked drug containers **(103).** In other embodiments, more or fewer linked drug containers **(103)** can be present. The linked drug containers **(103)** are sealed by frangible membranes. A single frangible membrane can seal the top of one drug container **(103)** and the bottom of the adjacent drug container **(103),** separate frangible membranes **(103)** can be used for adjacent ends of drug containers, and any combination thereof. When the device is activated, compressed gas (curved arrows at bottom) enters the capsule **(10).** The pressure bursts the membranes, allowing mixing and exit into the nosepiece of the substance s within the linked drug containers **(103).**

In a preferred embodiment, each drug containers **(103)** is made of a soft thin sheet. The sheet can be a polymeric membrane, a continuous sheet or any other form which is thin enough to be easily torn when desired by the released of the compressed air. All drug containers **(103)** are connected to each other during manufacturing. Mixing occurs only during activation, with the compressed gas tearing the membranes/sheets dividing the compartments. Once the membranes are torn, the substance s are exposed to the compressed gas, mixed and delivered.

**Fig. 17D** shows an embodiment with sets of two-layer membranes **(104A, 104B)** within the capsule **(10).** In this exemplary embodiment, there are 4 sets of two-layer membranes **(104A, 104B).** In other embodiments, more or fewer sets of two-layer membranes **(104A, 104B)** can be present. The lower membrane **(104B)** is reticulated, with portions separable from each other, and the upper membrane **(104A),** frangible. When the device is activated, compressed gas (curved arrows at bottom) enters the capsule **(10).** The pressure causes the separable portions of the lower membrane **(104B)** to rotate upward, tearing the upper membrane **(104A)** and allowing mixing and exit into the nosepiece of the substance s within the capsule **(10).**

This embodiment differs from the previous one in that: (a) the drug containers do not form one unit; (b) the separate zones are separated from each other by membrane which is composed of two layers: one provides the rigidity of the membrane and is made of a rigid material, and the other one is a continuous flexible sheet which seals against the lower rigid part during until activation and which opens when air is pressed against its lower side The membranes **(104A, 104B)** open only one way, when air presses against their lower side during activation, allowing mixing of the substances during delivery.

**Fig. 17E** shows an embodiment with duckbill valves **(105)** within the capsule **(10).** In this exemplary embodiment, there are 4 duckbill valves **(105).** In other embodiments, more or fewer duckbill valves **(105)** can be present. When the device is activated, compressed gas (curved arrows at bottom) enters the capsule **(10).** The pressure causes the duckbill valves **(105)** to rotate upward, allowing exit and mixing of the substance s within the capsule **(10).**

**Fig. 17F** shows an embodiment with frangible membranes **(105)** within the capsule **(10).** In this exemplary embodiment, there are 4 frangible membranes **(105).** In other embodiments, more or fewer frangible membranes **(105)** can be present. When the device is activated, compressed gas (curved arrows at bottom) enters the capsule **(10).** The pressure causes the frangible membranes **(105)** to tear, allowing mixing and exit into the nosepiece (not shown) of the substance s within the capsule **(10).**

**Fig. 17G** shows an embodiment with bendable membranes **(106)** within the capsule **(10).** In this exemplary embodiment, there are 4 bendable membranes **(106).** In other embodiments, more or fewer bendable membranes **(106)** can be present. When the device is activated, compressed gas (curved arrows at bottom) enters the capsule **(10).** The pressure causes the bendable membranes **(106)** to rotate upward (curved arrows in middle) about connection points between the bendable membranes **(106)** and the capsule **(10)** wall, allowing mixing and exit into the nosepiece (not shown) of the substances within the capsule **(10).**

These exemplary embodiments allow holding the substances separate during storage and mixing the substances only upon activation and delivery. In some embodiments, the device or the substances therein can be configured to generate a temperature change, either heating or cooling, during mixing and delivery. The device can further be configured so that components for creating a temperature change in the device are not released with the delivered substances.

Heating and cooling can be triggered by mechanical force, by pressure, by chemical reaction and any combination thereof. This can be done inside the drug capsule, around the drug capsule, or outside the device itself in its packaging, to be triggered right before activation of the device.

Such temperature change can be generated during activation (short time temperature change) or prior to activation (long time temperature change). Long time temperature changes require a temperature activation separated from the delivery activation.

Either option, or at least the long time temperature change, further requires proper device sealing to allow temperature to be maintained inside the device and to allow equilibration prior delivery. Such options can further include a temperature indicator, such as by a color change in a dedicated control window, to allow the user to know that the device is ready for activation.

A temperature change can be an increase in temperature, a decrease of temperature, or both.

A temperature change can be useful for example for:
- Substance mixing
- Dissolution of one substance in another
- Absorption of a substance or mixture of substances in tissue, for example, a delivery temperature regulated with respect to the temperature of the nasal passages.
- Effective scattering of a substance or mixture of substances on tissue, for example, to create a flat, thin, uniform layer in the nasal passages and hence improve absorption
- Affect the viscosity of a substance or mixture of substances (both increase and decrease of viscosity can occur).
- Affect nature of a substance or mixture of substances. For example: polymerization can be initiated only during delivery, or during or after contact with tissue.

One embodiment comprises two heating agents. These heating agents are in compartments of a capsule. Upon activation of the device, or upon activation of heating (for example, buy pressing a button), a membrane separating the two compartments is torn, allowing the heating agents to mix and to generate heat within the device. Other membranes are not torn by this activity, which keeps the heating agents in a sealed compartment - sealed so as to prevent delivery of heating agent delivery but allow gas passage to other compartments. Passage of the compressed gas then delivers the heated substances or other desired substances. Mixing, as disclosed above, can occur during delivery.

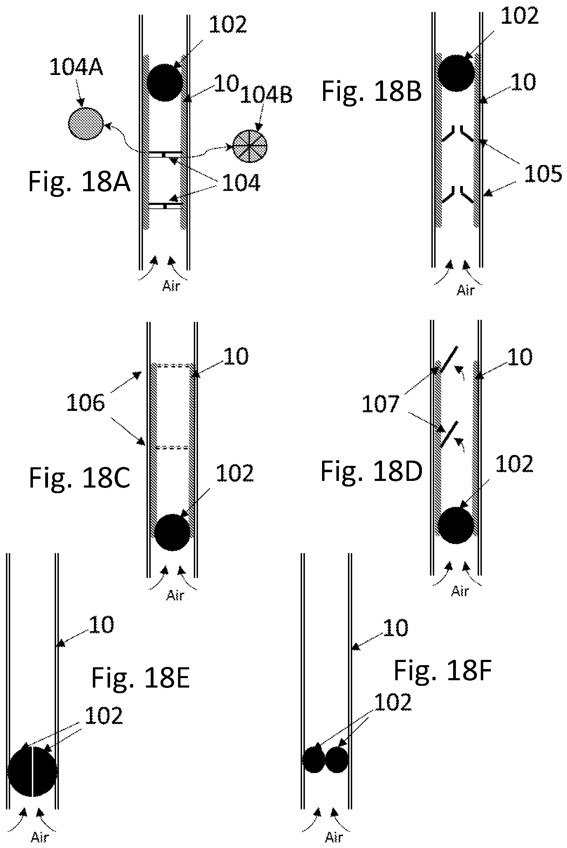

Reference is now made to **Figs. 18A-18F** illustrating another embodiment of the capsule.

**Fig. 18A** shows an embodiment with sets of two-layer membranes **(104A, 104B)** and a mixing ball **(102)** within the capsule **(10).** In this exemplary embodiment, there are 2 sets of two-layer membranes **(104A, 104B)** and a single mixing ball **(102)** at the top of the capsule **(10).** In other embodiments, more or fewer sets of two-layer membranes **(104A, 104B)** and more or fewer mixing balls **(102)** can be present; the mixing balls **(102)** can be at any desired location within the capsule **(10).** The lower membrane **(104B)** is reticulated, with portions separable from each other, and the upper membrane **(104A),** frangible. When the device is activated, compressed gas (curved arrows at bottom) enters the capsule **(10).** The pressure causes the separable portions of the lower membrane **(104B)** to rotate upward, tearing the upper membrane **(104A)** and allowing mixing and exit into the nosepiece of the substances within the capsule **(10).** Further mixing is provided by the mixing ball **(102).** As disclosed above, a shoulder or other stopper in the nosepiece (not shown) prevents the mixing ball **(102)** from exiting the nosepiece (not shown).

**Fig. 18B** shows an embodiment with duckbill valves **(105)** and a mixing ball **(102)** within the capsule **(10).** In this exemplary embodiment, there are 2 duckbill valves **(105)** and a single mixing ball **(102)** at the top of the capsule **(10).** In other embodiments, more or fewer duckbill valves **(105)** can be present and more or fewer mixing balls **(102)** can be present; the mixing balls **(102)** can be at any desired location within the capsule **(10).** When the device is activated, compressed gas (curved arrows at bottom) enters the capsule **(10).** The pressure causes the duckbill valves **(105)** to rotate upward, allowing exit and mixing of the substances within the capsule **(10).** Further mixing is provided by the mixing ball **(102).** As disclosed above, a shoulder or other stopper in the nosepiece (not shown) prevents the mixing ball **(102)** from exiting the nosepiece (not shown).

**Fig. 18C** shows an embodiment with frangible membranes **(105)** and a mixing ball **(102)** within the capsule **(10).** In this exemplary embodiment, there are 4 frangible membranes **(105)** and a single mixing ball **(102)** at the top of the capsule **(10).** In other embodiments, more or fewer frangible membranes **(105)** can be present and more or fewer mixing balls **(102)** can be present; the mixing balls **(102)** can be at any desired location within the capsule **(10).** When the device is activated, compressed gas (curved arrows at bottom) enters the capsule **(10).** The pressure causes the frangible membranes **(105)** to tear, allowing mixing and exit into the nosepiece (not shown) of the substances within the capsule **(10).** Further mixing is provided by the mixing ball **(102).** As disclosed above, a shoulder or other stopper in the nosepiece (not shown) prevents the mixing ball **(102)** from exiting the nosepiece (not shown).

**Fig. 18D** shows an embodiment with bendable membranes **(106)** and a mixing ball **(102)** within the capsule **(10).** In this exemplary embodiment, there are 4 bendable membranes **(106)** and a single mixing ball **(102)** at the top of the capsule **(10).** In other embodiments, more or fewer bendable membranes **(106)** can be present and more or fewer mixing balls **(102)** can be present; the mixing balls **(102)** can be at any desired location within the capsule **(10).** When the device is activated, compressed gas (curved arrows at bottom) enters the capsule **(10).** The pressure causes the bendable membranes **(106)** to rotate upward (curved arrows in middle) about connection points between the bendable membranes **(106)** and the capsule **(10)** wall, allowing mixing and exit into the nosepiece (not shown) of the substances within the capsule **(10).** Further mixing is provided by the mixing ball **(102).** As disclosed above, a shoulder or other stopper in the nosepiece (not shown) prevents the mixing ball **(102)** from exiting the nosepiece (not shown).

**Fig. 18E** shows an embodiment with two half balls **(102).** In this exemplary embodiment, there is one pair of half-balls **(102).** In other embodiments, more pairs of half-balls **(102)** can be present. When the device is activated, compressed gas (curved arrows at bottom) enters the capsule **(10).** The pressure causes the half-balls **(102)** to move upward. They will separate and tumble as they move, allowing gas to pass between and around them thus mixing and delivering the substance. As disclosed above, a shoulder or other stopper in the nosepiece (not shown) prevents the mixing ball **(102)** from exiting the nosepiece (not shown).

**Fig. 18F** shows an embodiment with two attached mixing balls **(102).** In other embodiments, more mixing balls **(102)** can be present. When the device is activated, compressed gas (curved arrows at bottom) enters the capsule **(10).** The pressure causes the mixing balls **(102)** to move upward, thus causing efficient mixing of the substances. As disclosed above, a shoulder or other stopper in the nosepiece (not shown) prevents the mixing ball **(102)** from exiting the nosepiece (not shown).

The mixing balls need not be spherical; any shape that will provide good sealing during storage and low-friction movement during activation can be used.

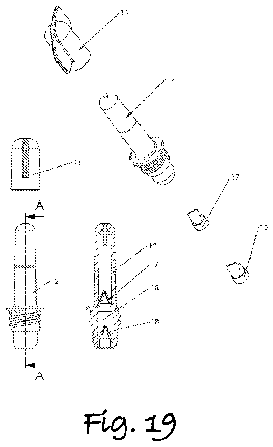

Reference is now made to **Fig. 19****,** which illustrates a closer view of another embodiment of the capsule. According to this embodiment, the capsule comprises at least one uni-directional valve such as duckbill valve. In the embodiment illustrated in **Fig. 19** two unidirectional valve (e.g., duckbill valves), 17 and 18 are provided, where the medicament 15 is disposed therebetween.

As noted above, the medicament delivered can either powder, liquids, gas, gel and any mixing thereof.

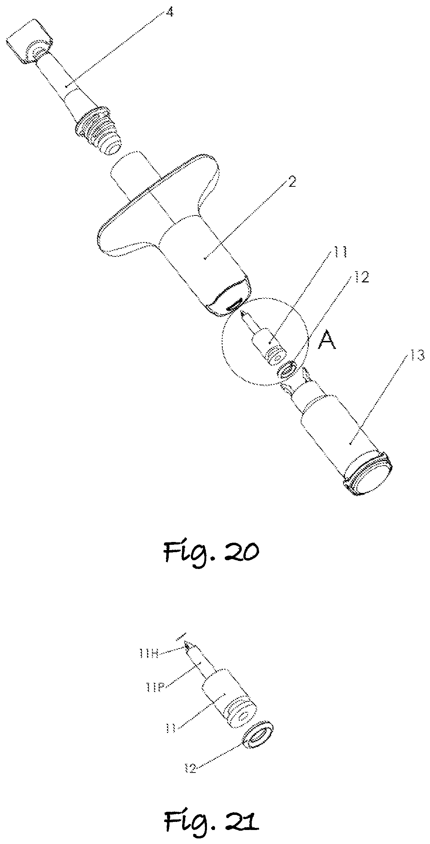

Reference is now made to **Figs. 20-21** which illustrates another embodiment of the present technology in which piercing of the medicament container is disclosed. **Fig. 20** illustrates the device according to this embodiment, where 4 is the nosepiece, 2 is the device's body, 11 is a hollow piercing element, 13 is the pressurized gas container and 12 is a sealing element (preferably an o-ring) sealing the pressurized gas container 13.

**Fig. 21** illustrates a closer view of the hollow piercing element 11. According to this embodiment the hollow piercing element 11 comprising (a) a hollow sharp end 11P having at least one orifice 11H (throughout which the pressurized gas will exit the hollow piercing element 11 and enter the medicament container, as will be disclosed hereinafter); and, (b) a proximal end (close to the pressurized gas container). According to one embodiment, the proximal end of the hollow piercing element 11 has at least groove along the circumference thereof upon which at least one o-ring rests to provide sealing of the pressurized gas container 13. The two ends of the hollow piercing element 11 are interconnected by a hollow tube.

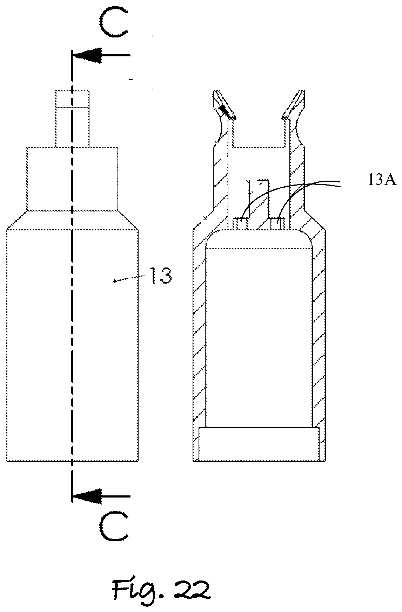

Reference is now made to **Fig. 22** which illustrates the pressurized gas container 13. According to this embodiment, the pressurized gas container 13 comprises at least one orifices 13A (throughout which the pressurized gas exits). In **Fig. 22****,** two orifices 13A are illustrated.

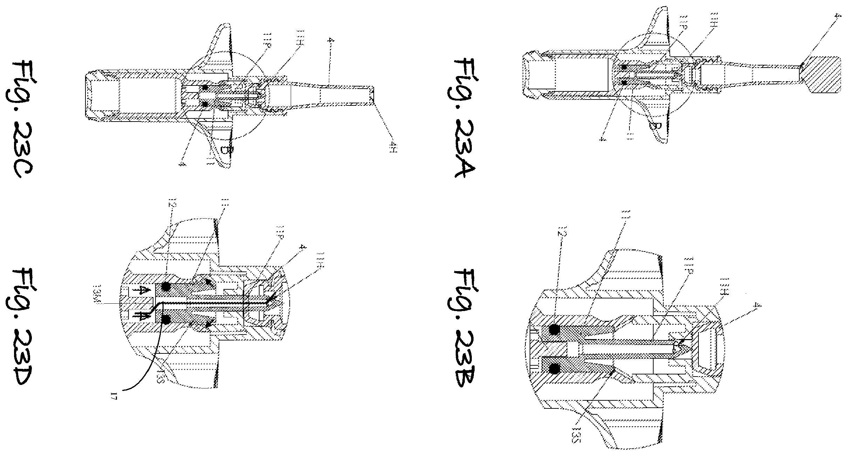

Reference is now made to **Figs. 23A-23D****,** which illustrate the mechanism of action of the device according to the present embodiment.

**Figs. 23A-23B** illustrate the device before activation (i.e. before the hollow piercing element 11 pierced the medicament container in the nosepiece 4). In the figures, the hollow piercing element 11 with the hollow sharp end 11P having at least one orifice 11H are shown. Also shown is the o-ring 12 disposed in the proximal end thereof. **Fig. 23B** is a closer view of the hollow piercing element 11 before piercing the nosepiece 4 and the medicament capsule therewithin. Before activation of the device (i.e., pressing on the pressurized gas container 13), the bottom end of the hollow piercing element 11 seals the orifices 13A of the pressurized gas container 13 and prevent release of said pressurized gas.

Numerical reference 13S illustrates a stopper element that ensures the hollow piercing element 11 is not piercing the medicament container before activation.

**Figs. 23C-23D** illustrate the device after activation (i.e. after the hollow piercing element 11 pierced the medicament container in the nosepiece 4). In the figures, after the pressurized gas container has been pressed, the hollow piercing element 11 has pierced the nosepiece 4, and the bottom end of the hollow piercing element 11 is moved from it position, such that the sealing of the orifices 13M of the pressurized gas container 13 is removed and the release of said pressurized gas from the pressurized gas container 13 is enabled through orifices 13A.

Once the pressurized gas exits from the orifices 13A, it enters the hollow tube of the hollow piercing element 11 and exits thereof from orifice 11H in the sharp end 11P into the medicament container in the nosepiece 4. The pathway of the pressurized air is illustrated by numerical reference 17.

**Fig. 23D** is a closer view of the hollow piercing element 11 after piercing the nosepiece 4 and the medicament capsule therewithin and pathway of the pressurized air exiting the orifices 13A pressurized gas container 13 and entering the medicament container.

According to this embodiment, illustrated in **Figs. 23A-23D****,** the capsule encloses a liquid medicament. However, as described above, also powder medicament are enabled and the mixing thereof.

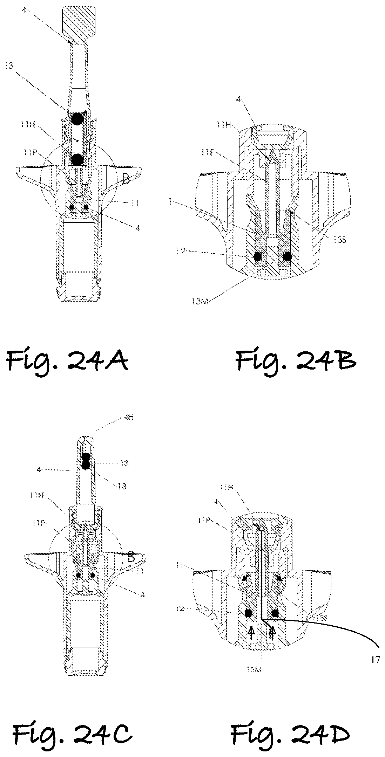

**Figs. 24A-24D** illustrates the piercing mechanism utilized with a powder medicament. According to this embodiment, the nosepiece encloses a capsule with at least one spherical element 13. In **Fig. 24A****,** two spherical elements 13 are illustrated, one at the bottom most part of the capsule and the other at the upper most part. The medicament is disposed therebetween.

As described above, after the pressurized gas container has been pressed, the hollow piercing element 11 has pierced the nosepiece 4, and the bottom end of the hollow piercing element 11 is moved from it position, such that the sealing of the orifices 13M of the pressurized gas container 13 is removed and the release of said pressurized gas from the pressurized gas container 13 is enabled through orifices 13A.

Once the pressurized gas exits from the orifices 13A, it enters the hollow tube of the hollow piercing element 11 and exits thereof from orifice 11H in the sharp end 11P into the medicament container in the nosepiece 4. Such pressurized gas removes the bottom most spherical element 18 from its position to compress the medicament until the pressure reaches the upper most spherical element to remove it from its position. Once the two spherical elements are removed, the medicament and the pressurized gas are mixed and delivered to the nasal cavity. **Fig. 24B** is a closer view of the hollow piercing element 11 before piercing the nosepiece 4.

Reference is now made to **Figs. 24C-24D** which illustrate the device after activation (i.e. after the hollow piercing element 11 pierced the medicament container in the nosepiece 4). As detailed above, once the pressurized gas container has been pressed, the hollow piercing element 11 has pierced the nosepiece 4, and the bottom end of the hollow piercing element 11 is moved from it position, such that the sealing of the orifices 13A of the pressurized gas container 13 is removed and the release of said pressurized gas from the pressurized gas container 13 is enabled through orifices 13A.

Once the pressurized gas exits from the orifices 13A, it enters the hollow tube of the hollow piercing element 11 and exits thereof from orifice 11H in the sharp end 11P into the medicament container in the nosepiece 4 to remove the spherical element/s. The pathway of the pressurized air is illustrated by numerical reference 17. **Fig. 24D** is a closer view of the hollow piercing element 11 after piercing the nosepiece 4 and the pressurized gas pathway.

It should be noted that a variety of capsule and sealing means (other than the spherical element) can be utilized. Several examples are provided in **Figs. 17A-17G** and **Figs. 18A-18F****.**

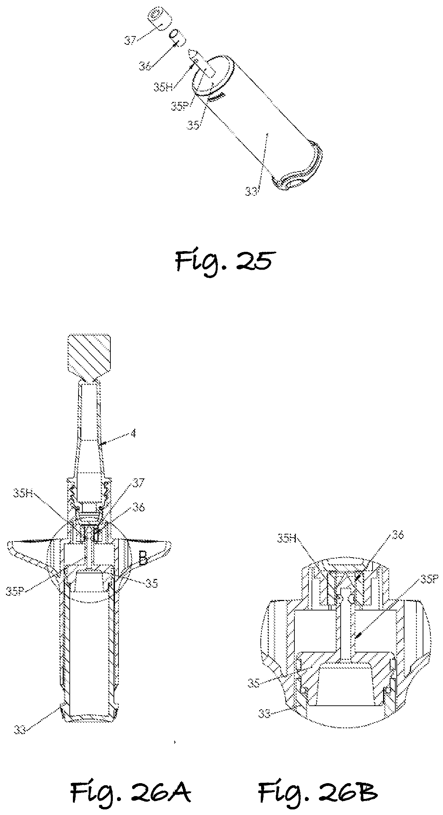

Reference is now made to **Figs. 25-30D** which illustrates another embodiment of the piercing mechanism.

According to this embodiment, the pressurized gas container 33 having an upper surface 35 integrated with a hollow extension body 35P and a sharp end with at least one orifice 35H along the body 35P or at the upper most end of the hollow extension 35. A septum 36 is provided to seal said at least one orifice 35H. An optional septum cover 37 is placed on the septum in a tight manner in order to insure sealing before activation.

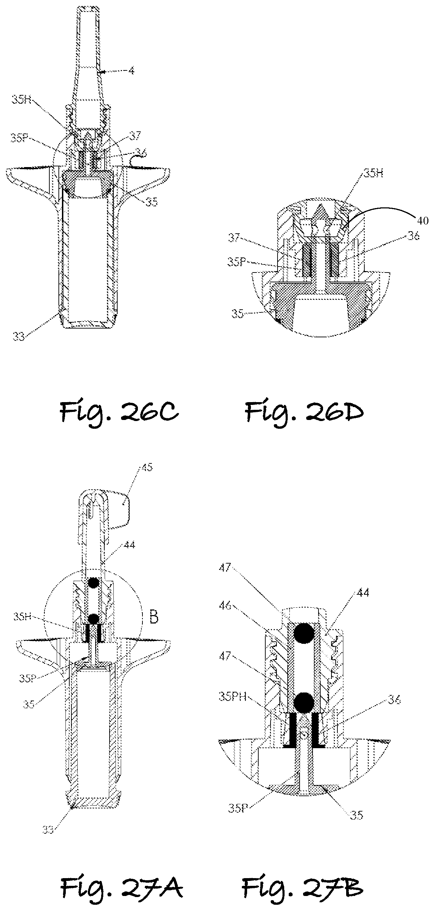

Reference is now made to **Figs. 26A - 26B** which illustrates the above disclosed embodiment, prior to activation. As can be seen from **Fig. 26A****,** prior to pressing on the pressurized gas container 33, the hollow extension 35 has not yet pierced the nosepiece 4 and the medicament container. **Fig. 26B** provides a closer view of the hollow extension 35. As can be seen from the figures, the orifice 35H of the hollow extension 35 is sealed with septum 36 and its optional cover 37, such that the pressurized gas from the pressurized gas container 33 is prevented from escaping therefrom.

Reference is now made to **Figs. 26C-26D** which the above disclosed embodiment, post to activation (i.e., after piercing of the nosepiece). As can be seen from the figures, once the pressurized gas container has been activated (i.e., pressed), the hollow extension 35 is pushed towards the nosepiece. Such movement results in: (a) piercing of the capsule within the nosepiece 4 by means of the sharp end of the hollow extension 35; and, (b) the hollow extension 35 moves through the septum 36 and its cover 37 (those are static in the device and can not move), and thus the orifice 35H is being exposed (uncovered) to enable a fluid communication between the pressurized gas container 33 and the capsule.

Reference is now made to **Figs. 27A-27D** illustrating the above embodiment utilizing a capsule 46 dedicated to either powder or liquid medicaments. In such capsule at least one spherical element 47 is utilized. The capsule 46 illustrated in **Figs. 27A-D** employs 2 spherical elements 47. One positioned at the bottom most part of the capsule and the other at the uppermost part. The medicament is positioned therebetween. **Figs. 27A -27B** illustrated this embodiment prior to activation of the device.

It should be noted that should the capsule enclose several spherical elements 47 the same can be used as divider to enclose several liquid medicaments, several powder medicaments or any combination of both liquid and powder medicaments. It is further noted that the spherical elements 47 can be replaced with a membrane adapted to divide the capsule to several 'compartments'.

It should further be noted that the hollow extension 35 can be used to remove the sealing of the capsule (in this case, the spherical element) or to pierce the capsule.

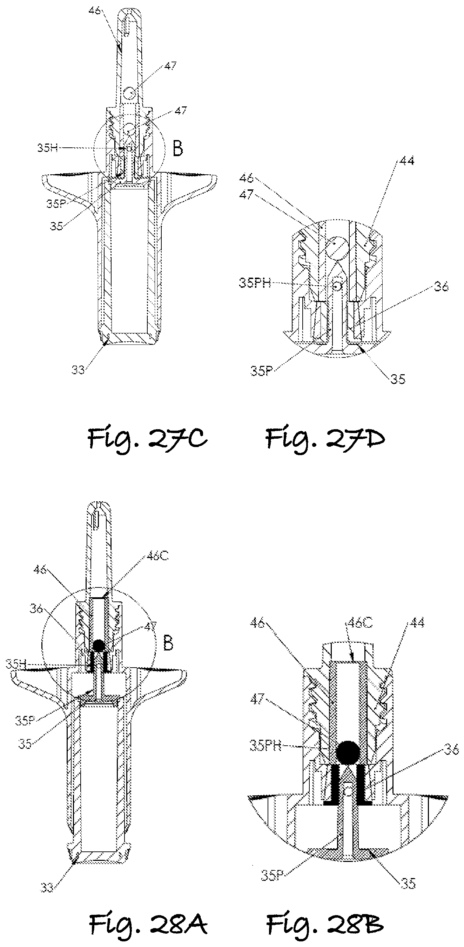

Reference is now made to **Figs. 27C-27D** illustrating said embodiment post activation (i.e., after the hollow extension 35 either pierced the nosepiece mechanically or released the compressed air in a manner that results in the opening of the barrier element in the capsule or nosepiece, such as the spherical element and /or membrane), to deliver a powder or liquid or any combination thereof medicament. Thus, it should be emphasized that it could very well be that the hollow extension 35 does not pierce the nosepiece but merely removes the sealing thereof (in this case the spherical element).

Once the pressurized gas container 33 is actuated (i.e., pressed), the hollow extension 35 is pushed towards the nosepiece. Such movement results in: (a) piercing of the capsule within the nosepiece 4 by means of the sharp end of the hollow extension 35; or removing the sealing element, which is, in this case, the spherical element; and, (b) the hollow extension 35 moves through the septum 36 and its cover 37 (those are static in the device and cannot move), and thus the orifice 35H is being exposed (uncovered) to enable a fluid communication between the pressurized gas container 33 and the capsule. In such a case, where powder or liquid medicament is utilized, the pressurized gas first remove bottom most spherical element 47, which in turn compress the medicament (positioned between the two spherical elements 47) until max. compression is reached and then the uppermost spherical element 47 is removed, where only then, the medicament and the pressurized gas come in contact, mix and being delivered. **Fig. 27D** provides a closer view of the piercing by means of hollow extension 35.

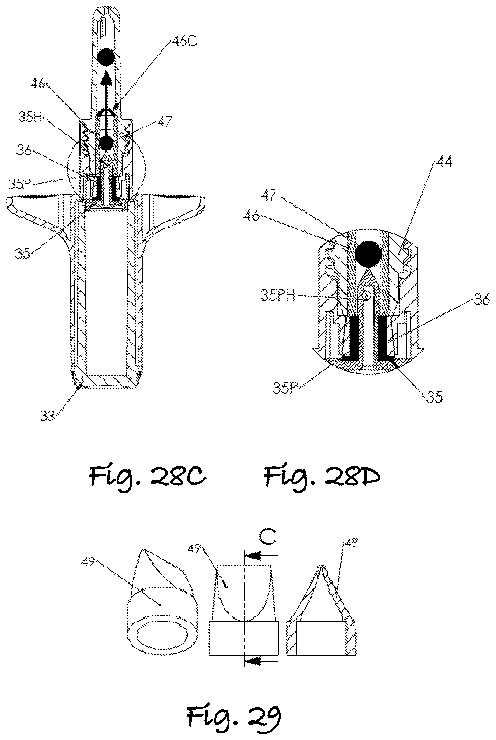

Reference is now made to **Figs. 28A, 28B****,** **28C, 28D** illustrating another embodiment as described in **Figs. 27A, 27B****,** **27C and 27D** but with one spherical element 47, positioned in the bottom most part of the capsule.

**Figs. 28A-28B** illustrates the device before activation and piercing of the capsule and **Figs. 28C-28D** illustrates the device after activation and piercing of the capsule 46 in the nosepiece 44. In the embodiment illustrated in **Fig. 28B** the capsule is also illustrated with a membrane 46C that enables the disposition of either liquids or powder medicaments (or any mix/combination thereof).

Reference is now made to **Fig. 29** illustrating one way valve embodied as a duckbill (similarly to **Fig. 19**).

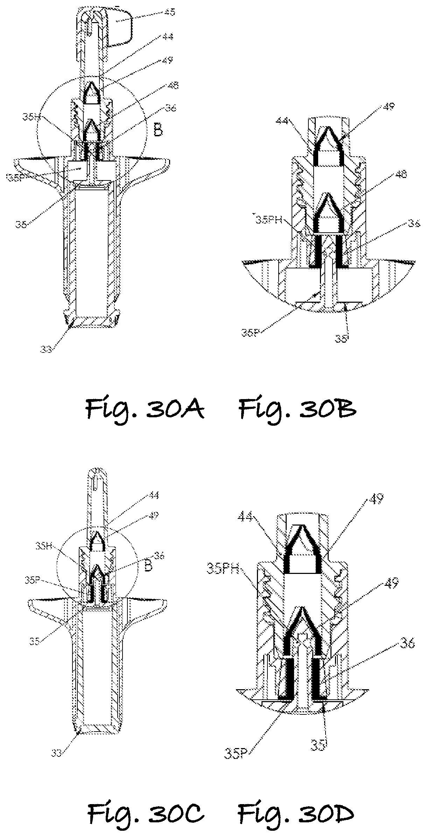

**Figs. 30A-30D** illustrating the device utilizing a unidirectional valve (such as a Duckbill) as the area to hold and store the drug (liquid/powder) prior to activation. Before the activation **(****Figs. 30A-30B****)** the drug is stored in a closed area between the valves (in some cases only one valve can be used). After activation **(****Figs. 30C-30D****),** the air flow from the pressurized air container is discharged trough the valve/s to release the drug via the nosepiece in the same manner as described for **Figs. 27-28****.**

It should be noted that the capsule could utilize several embodiments, e.g., the embodiments described in **Figs. 17A-17G** and **Figs. 18A-18F****.**

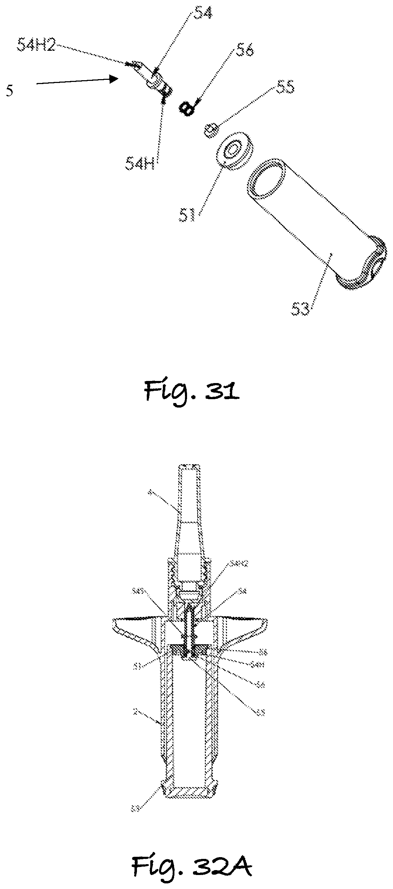

Reference is now made to **Fig. 31** which illustrates another embodiment of the present technology in which the hollow piercing element 5 which comprises (a) a hollow sharp end having at least one orifice 54H2 (throughout which the pressurized gas will exit the hollow piercing element 5 and enter the medicament container, as will be disclosed hereinafter); and, (b) a proximal end (close to the pressurized gas container). According to one embodiment, the proximal end of the hollow piercing element 5 has at least one groove along the circumference thereof upon which at least one o-ring 56 rests to provide sealing of the pressurized gas container 13 to prevent any pressurized gas from exiting the pressurized gas container 53 before activation thereof. The two ends of the hollow piercing element 5 are interconnected by a hollow tube 54.

According to one embodiment, the proximal end of the hollow piercing element 5 has at least one orifice 54H (throughout which the pressurized gas will exit the pressurized gas container 53 and enter the hollow piercing element 5, as will be disclosed hereinafter). Numerical reference no. 51 refers to the pressurized gas container 53's cover so as to prevent leakage therefrom. Numerical reference no. 55 is adapted to seal the bottom side of the hollow tube 54.

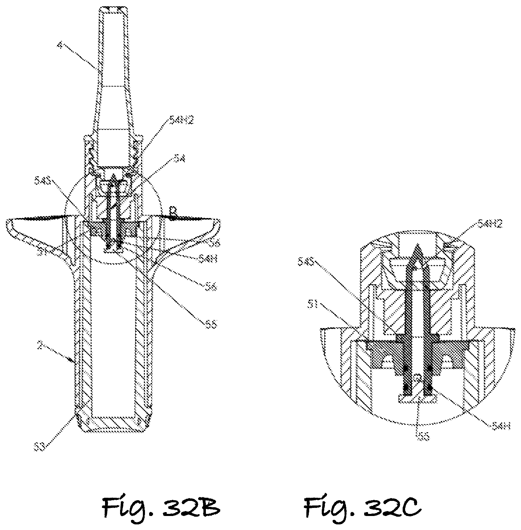

Reference is now made to **Fig. 32A** which illustrates the device before activation (i.e. before the hollow piercing element 5 pierced the medicament container in the nosepiece 4). As seen from the figure, before the bottom end of the pressurized gas container has been pressed the hollow piercing element 5 has not yet pierced the nosepiece 4. In this position, the at least one orifice 54H in the proximal end of the hollow piercing element 5 is placed above (i.e., outside) the pressurized gas container thus, maintain the pressurized gas container sealed. Furthermore, the at least one orifice 54H2 in the hollow sharp end of the hollow piercing element 5 is below (i.e., outside) the nosepiece.

Reference is now made to **Figs. 32B-32C** which illustrate the device after activation thereof. As detailed above, once the pressurized gas container has been pressed, the bottom most part 55 of hollow piercing element 5 is now positioned within the pressurized gas container. In this position, the orifice 54H (in the proximal end thereof) is disposed within the pressurized gas container thus, pressurized fluid (gas) can exit the pressurized gas container and enter the hollow piercing element 5 through orifice 54H. Substantially at the same, the sharp end of the hollow piercing element 5 pierced the nosepiece 4, and at least one orifice 54H2 is now disposed within the nosepiece (and the medicament capsule). Thus, the pressurized fluid (gas) can exit the hollow tube 54 of the hollow piercing element 5 from at least one orifice 54H2 and enter the nosepiece (and the medicament capsule) to entrain the medicament and deliver the same to the nasal cavity.

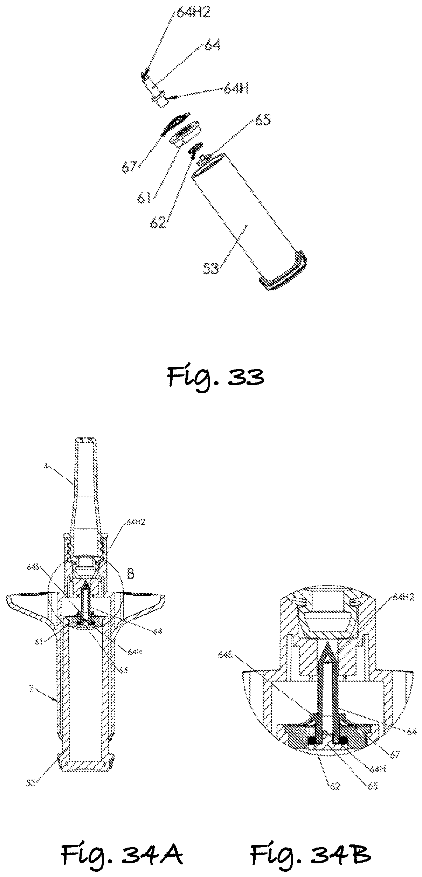

Reference is now made to **Figs. 33-34D** illustrating another embodiment of the present technology in which a spring 67 is utilized. In this embodiment, the hollow needle 64 has at least one orifice 64H2 in the upper end thereof, adapted to enable the release of the medicament to the nose. Needle 64 has at least one orifice 64H at the bottom most part thereof. Throughout which the pressurized gas exits the pressurized gas container and enter the hollow needle to eventually enter the capsule and entrain the medicament to be delivered to the nasal cavity.

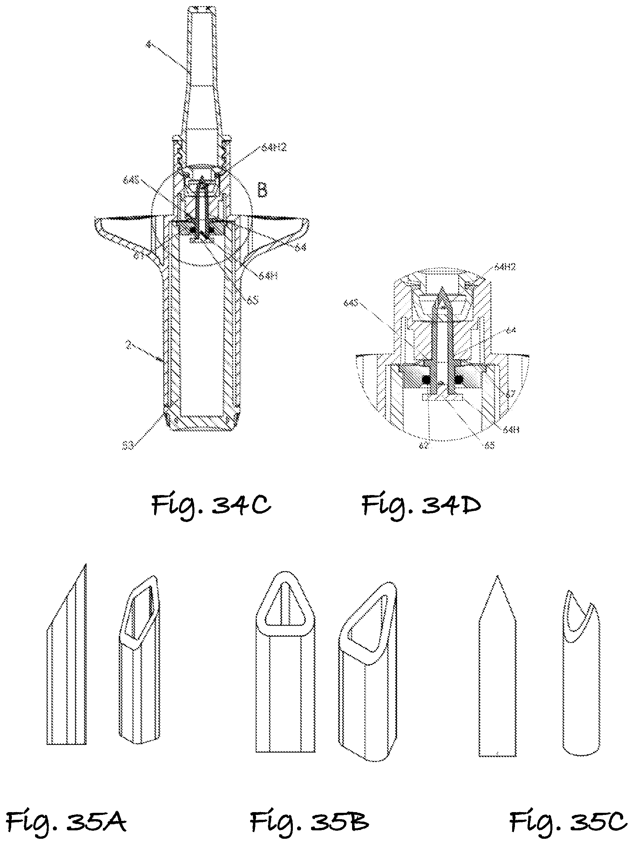

**Figs. 34A-34B** illustrates the device before activation (where **Fig. 34B** provides a closer view of the needle 64) and **Figs. 34C-34D** illustrates the device before activation (where **Fig. 34D** provides a closer view of the needle 64).

As seen in **Figs. 34A-34B****,** before activation of the device, orifice 64H is outside the pressurized gas container 53 and prevents the pressurized gas from exiting therefrom; and orifice 64H2 is outside the capsule in the nosepiece.

Once the bottom most part of the pressurized gas has been pressed, shoulders 64s presses spring 67 and the orifice 64H on the bottom of the needle being exposed. Furthermore, orifice 64H2 penetrates the capsule to enable the exit of the pressurized air from the hollow needle 64 to the capsule.

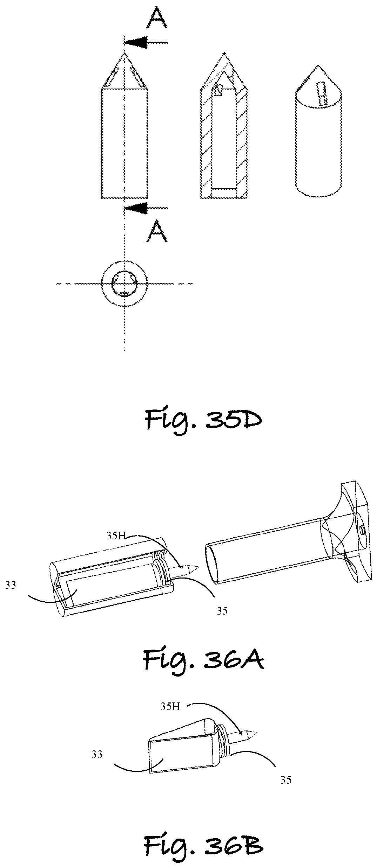

Reference is now made to **Figs. 35A-35D** illustrating different shapes of hollow needle. As can be seen from the figures, the sharp end of the needle can have a cross section of any shape (e.g., rectangular, triangular, pentagon etc.). Each wig of the needle can accommodate at least one orifice, throughout which the pressurized gas can exit and enter the capsule and entrain the medicament to be delivered to the nasal cavity.

According to any of the embodiment as described above, the pressurized gas container can be mad of at least one material selected from a group consisting of high- or low-density polyethylene, high- or low-density polypropylene, any plastic resin, glass, plastics, Aluminum and any combination thereof.

Reference is now made to **Figs. 36A-36B****,** illustrating another embodiment of the present technology, in which the pressurized-fluid container (enclosing the pressurized and compressed fluid) is made of material being high barrier film. According to one embodiment the high barrier film is high barrier Aluminum film.

According to this embodiment, the high barrier film (e.g., Aluminum film) is ultrasonic soldered to create the pressurized-fluid container. Such ultrasonic soldering of the Aluminum high barrier film results in an airtight container that prevents any air leakage of the compressed and pressurized gas therewithin.

Reference is now made to **Fig. 36A** which illustrates the container 33 (ultrasonic soldering) along with the upper surface 35 integrated with a hollow extension body 35P (not shown) and a sharp end with at least one orifice 35H along the body 35P or at the upper most end of the hollow extension 35. **Fig. 36B** illustrates a closer view thereof.

It should be emphasized that surface 35 is sealable welded to the upper surface of the container.

The invention, including preferred embodiments, have been presented for the purpose of illustration and description. They are not intended to be exhaustive or to limit the invention to the precise form disclosed. Obvious modifications or variations are possible in light of the above teachings. The embodiments were chosen and described to provide the best illustration of the principals of the invention and its practical application, and to enable one of ordinary skill in the art to utilize the invention in various embodiments and with various modifications as are suited to the particular use contemplated. All such modifications and variations are within the scope of the invention as determined by the appended claims when interpreted in accordance with the breadth they are fairly, legally, and equitably entitled.

## Claims

1. A device for delivering either one or more substances within at least one body cavity, comprising:
(i) at least one nosepiece (4) comprising at least one capsule comprising V_{sub} [ml or mg] of said one or more substances; said at least one capsule having at least one fluid inlet port of diameter Dᵢₙ [mm] and at least one fluid discharging outlet of diameter Dₒᵤₜ [mm], configured for placement in proximity to said at least one body cavity;
(ii) at least one pressurized gas container (13) in communication with said at least one nosepiece (4), said at least one pressurized gas container (13) comprises at least one chamber configured to confine compressed and pressurized fluid at volume V_{PF} [ml] and pressure P_{PF} [barg] and wherein said at least one pressurized gas container comprises at least one orifice (13A);
(iii) at least one hollow puncturing member (11); said at least one hollow puncturing member (11) is **characterized by** at least one sharp end (11P) and a second end; said second end being in fluid communication with said at least one pressurized gas container (13) and sealing the at least one orifice (13A) of the at least one pressurized gas container (13); said first and second ends are fluidly interconnected to each other by at least one hollow tube;
said at least one fluid inlet port of said at least one capsule is configured by means of size and shape to interface in a sealable manner with said at least one sharp end (11P) of said at least one hollow puncturing member (11);
wherein actuation of said at least one pressurized gas container (13) is configured to enable piercing of said at least one capsule to provide said at least one fluid inlet port, by means of said at least one sharp end (11P) of said puncturing member (11), and pressing on the at least one pressurized gas container (13) results in a relative movement of the second end of the at least one hollow puncturing member (11) from its position, such that the sealing of the at least one orifice (13A) of the at least one pressurized gas container is removed, to enable the pressurized fluid to exit said at least one chamber and enter through a hollow tube of said at least one hollow puncturing member (11) to said at least one capsule, entrain said one or more substances and deliver the same to said at least one body cavity.

2. The device of claim 1, wherein said at least one chamber is made of material being high barrier film; wherein said high barrier film is high barrier Aluminum film.

3. The device of claim 1, wherein actuation of said at least one pressurized gas container (13) comprises pressing on the same.

4. The device of claim 1, wherein said second end of said at least one hollow puncturing member (11) and said at least one chamber are formed as a single unit, such that the upper surface (35) of said at least one chamber is integrated with said second end of said at least one hollow puncturing member (11).

5. The device of claim 1, wherein said at least one sharp end (11P) of said at least one hollow puncturing member (11) comprises at least one second orifice throughout which said pressurized fluid enters said at least one capsule.

6. The device of claim 5, additionally comprising at least one sealing septum, adapted to, prior to actuation of said at least one pressurized gas container (13), circumference and seal said at least one second orifice in said at least one sharp end (11P) of said at least one hollow puncturing member (11).

7. The device of claim 6, wherein upon actuation of said device, said at least one sealing septum is removed to enable exit of said pressurized fluid throughout said at least one second orifice in said at least one hollow puncturing member (11).

8. The device of claim 7, wherein upon actuation of said device, said at least one pressurized gas container (13) is pressed and said at least one hollow puncturing member (11) is pushed towards said at least one nosepiece (4) such that the bottom most part of said at least one nosepiece (4) removes said sealing septum from circumferencing said at least one second orifice in said at least one sharp end (11P) of said at least one hollow puncturing member (11).

9. The device of claim 8, wherein said at least one sharp end (11P) of said at least one hollow puncturing member (11) comprises at least one second orifice, such that, once said device is actuated, said pressurized fluid enters said at least one capsule through said at least one second orifice of said at least one hollow puncturing member (11).

10. The device of claim 1, wherein said at least one chamber comprises said at least one orifice (13A) throughout which said pressurized fluid exits said at least one chamber; further wherein at least one of the following is held true: (a) said second end of said at least one hollow puncturing member (11) seals said at least one orifice (13A) of said at least one chamber, prior to actuation of said device; (b) upon actuation of said device said sealing of said at least one orifice (13A) of said at least one chamber is removed, such that said pressurized fluid exits through said at least one orifice (13A) of said at least one chamber into said hollow tube of said at least one hollow puncturing member (11); (c) and any combination thereof.

11. The device of claim 10, wherein said at least one sharp end (11P) of said at least one hollow puncturing member (11) comprises at least one second orifice, such that, once said device is actuated, said pressurized fluid enters said at least one capsule through said at least one second orifice of said at least one hollow puncturing member (11).

12. The device of claim 1, wherein at least one of the following is held true (a) said at least one nosepiece (4) comprising at least one nosepiece (4) cover configured by means of size and shape to cover, in a sealable manner, at least partially said at least one nosepiece (4); and wherein said at least one nosepiece cover and said at least one nosepiece (4) are coupled to each other; (b) said at least one nosepiece (4) comprises at least one port throughout which said at least one substance exits said device, such that said at least one nosepiece (4) cover seals said at least one port and removal thereof removes said seal; (c) wherein said at least one capsule is selected from a group consisting of pierceable container (2), a blow-fill-seal and a form-fill-seal and any combination thereof; (d) said at least one chamber is a container (2) adapted to hold said pressured fluid at said P_{PF} for a period of time; (e) said at least one capsule is made of at least one material selected from a group consisting of high- or low-density polyethylene, high- or low-density polypropylene, any plastic resin, glass and any combination thereof; (f) said at least one chamber is configured to accept said volume V_{PF} [ml] of said pressurized fluid at pressure P_{PF} [barg] and configured such that said pressurized fluid is released from said at least one chamber within a short period of time, <500 milliseconds (dT), via said at least one fluid inlet port, entrains said substances, erupts via said at least one fluid discharging outlet port into said at least one body cavity, such that the release time of said V_{sub} [ml or mg] of said substances and said V_{PF} [ml] of said pressurized fluid, dTᵣₑₗₑₐₛₑ is less than 500 milliseconds; (g) any combination thereof.

13. The device of claim 1, wherein said device is configured to deliver said V_{sub} substance and V_{PF} pressurized fluid through said at least one fluid discharging outlet of diameter D [mm] wherein at least one of the following is held true:
a. V_{PF} is in a range of 1 to 50 ml;
b. V_{sub} is in a range of about 0.01 to about 7 ml;
c. Dᵢₙ and/or Dₒᵤₜ is in a range of 0.2 to 6 mm;
d. P_{PF} is in a range of about 0 to about 10 barg;
e. a pressure velocity is greater than 0.001 barg /ms;
f. said pressure velocity is greater than 0.01 barg /ms;
g. a volume rate dV_{sub}/dT or dV_{sub}/dTᵣₑₗₑₐₛₑ is greater than 0.0001 ml /ms;
h. said volume rate dV_{sub}/dT or dV_{sub}/dTᵣₑₗₑₐₛₑ is greater than 0.001 ml /ms;
i. said volume rate dV_{PF}/dT or dV_{PF}/dTᵣₑₗₑₐₛₑ is greater than 0.001 ml /ms;
j. said volume rate dV_{PF}/dT or dV_{PF}/dTᵣₑₗₑₐₛₑ is greater than 0.01 ml /ms;
k. any combination thereof.

14. The device of claim 1, wherein at least one of the following is true:
a. said at least one body cavity is selected from a group consisting of nasal cavity, the mouth, the throat, an ear, the eye, the vagina, the rectum, the urethra, and any combination thereof.
b. said pressurized gas is selected from a group consisting of air, nitrogen, oxygen, carbon dioxide, helium, neon, xenon, nitric oxide and any combination thereof;
c. during dispensing of said at least one substance, a mixture of said predetermined volume V_{gas} [ml] of said pressurized gas with said predetermined volume V_{sub} [ml or mg] of said substance entrained within it forms a plume of aerosol (718); said aerosol (718) having a predetermined distribution, said distribution being either homogeneous or heterogeneous, said heterogeneous distribution is selected from a group consisting of: an arbitrary distribution, a distribution in which the density of said at least one substance within said mixture follows a predetermined pattern, and any combination thereof; characteristics of said aerosol (718) selected from a group consisting of: particle size, particle shape, particle distribution, and any combination thereof, are determinable from characteristics of said device selected from a group consisting of: said predetermined volume of said pressurized gas, said predetermined volume of said substance, said predetermined pressure of said pressurized gas, said predetermined orifice size, and any combination thereof;
d. at least one of said substance is selected from a group consisting of a gas, a liquid, a powder, an aerosol (718), a slurry, a gel, a suspension and any combination thereof;
e. at least one said substance is stored under either an inert atmosphere or under vacuum to prevent reactions during storage;
f. a dose-response curve is substantially linear for brain concentration of said substance when administered nasally via said device; and
g. a dose-response curve for brain concentration having a fit selected from a group consisting of logarithmic, parabolic, exponential, sigmoid, power-low, and any combination thereof; of said substance when administered nasally via said device.

15. The device of claim 1, wherein at least one of the following is being held true (a) said at least one nosepiece (4) cover configured to provide an air-tight closure for said at least one port, said port cover slidable along said device, rotatable around said device, rotatable around a hinge on the exterior of said device and any combination thereof; (b) said device is configured such that said pressurized fluid entrains said substance in a pulsed manner, such that a plurality of potions V_{PF} erupts via said at least one fluid discharging outlet to within said at least one body cavity; (c) said device further comprises a safety latch (720), adapted to prevent accidental operation of said device; (d) said substance is selected from a group consisting of proteins; stem-cells; cells, organs, portions, extracts, and isolations thereof; macro-molecules; RNA or other genes and proteins-encoding materials; neurotransmitters; receptor antagonists; hormones; Ketamine; Baqsimi product commercially available by Lilly (US); Glucagon; substrates to treat one of the followings: anaphylaxis, Parkinson, seizures and opioid overdose; epinephrine; atropine; metoclopramide; commercially available Naloxone or Narcan products; Esketamine (Spravato); Radicava [edaravone]; Ingrezza [valbenazine]; Austedo [deutetrabenazine]; Ocrevus [ocrelizumab]; Xadago [safinamide]; Spinraza [nusinersen]; Zinbryta [daclizumab]; Nuplazid [pimavanserin]; Aristada [aripiprazole lauroxil]; Vraylar [cariprazine]; Rexulti [brexpiprazole]; Aptiom [eslicarbazepine acetate]; Vizamyl [flutemetamol F18 injection]; Brintellix [vortioxetine]; Tecfidera [dimethyl fumarate]; Dotarem [gadoterate meglumine]; Antibody mediated brain targeting drug delivery including aducanumab, gantenerumab, bapineuzumab, solanezumab, ofatumumab CD20, BIIB033, LCN2, HMGB1; insulin; oxytocin; orexin-A; leptin; benzodiazepine, midazolam; naloxone; perillyl alcohol; camptothecin; phytochemicals including curcumin and chrysin; nucleotides; olanzapine; risperidone; Venlafaxin; GDF-5; zonisamide; ropinirole; plant-originated and synthetically-produced terpenes and cannabinoids, including THC and CBD; valproric acid; rivastigmine; estradiol; topiramate or an equivalent preparation comprising CAS No. 97240-79-4; MFSD2 or MFSD2A or sodium-dependent lysophosphatidylcholine symporter, midazolam; naloxone; perillyl alcohol; camptothecin; phytochemicals including curcumin and chrysin; nucleotides; olanzapine; risperidone; Venlafaxin; GDF-5; zonisamide; ropinirole; plant-originated and synthetically-produced terpenes and cannabinoids, including THC and CBD; valproric acid; rivastigmine; estradiol; topiramate or an equivalent preparation comprising CAS No. 97240-79-4; MFSD2 or MFSD2A or sodium-dependent lysophosphatidylcholine symporter; and any esters, salts, derivatives, mixtures, combinations thereof, with or without a carrier, liposomes, lyophilic or water-miscible solvents, surfactants, cells, cells fractions, at a therapeutically effective concentration; (e) any combination thereof.

16. The device of claim 1, wherein said at least one capsule is a hollow tube (54) **characterized by** at least two ends interconnect to each other, at least one of which is positioned proximal to said at least one chamber.

17. The device of claim 16, wherein at least one of the following is held true (a) said at least one capsule comprises at least one spherical element positioned at at least one of said ends, adapted to seal said at least one capsule and prevent leakage of said at least one substrate therefrom; (b) said at least one capsule comprises two spherical elements, each of which is disposed at each of said ends, such that said at least one substrate is position therebetween; wherein said two spherical elements, once said pressurized fluid exits said at least one chamber, are adapted to mix said at least one substrate and said at least one substrate; (c) said at least one capsule comprises at least one membrane positioned at at least one of said ends, adapted to seal said at least one capsule and prevent leakage of said at least one substrate therefrom; wherein said at least one capsule comprises two membranes, each of which is disposed at each of said ends, such that said at least one substrate is position therebetween; (d) said at least one capsule comprises at least one duckbill valve positioned at at least one of said ends, adapted to seal said at least one capsule and prevent leakage of said at least one substrate therefrom; wherein said at least one capsule comprises two duckbill valves, each of which is disposed at each of said ends, such that said at least one substrate is position therebetween; (e) wherein said at least one capsule comprises at least one spherical element, membrane, uni-directional valve, duckbill valve and any combination thereof; (f) any combination thereof.

## Patentansprüche

1. Vorrichtung zur Abgabe einer oder mehrerer Substanzen in mindestens eine Körperhöhle, aufweisend:
(i) mindestens ein Nasenelement (4), das mindestens eine Kapsel aufweist, die V_{sub} [ml oder mg] der einen oder mehreren Substanzen enthält; wobei die mindestens eine Kapsel mindestens eine Fluideinlassöffnung mit einem Durchmesser Dᵢₙ [mm] und mindestens eine Fluidauslassöffnung mit einem Durchmesser Dₒᵤₜ [mm] aufweist, die zum Platzieren in der Nähe der mindestens einen Körperhöhle konfiguriert ist;
(ii) mindestens einen Druckgasbehälter (13), der mit dem mindestens einen Nasenelement (4) in Verbindung steht, wobei der mindestens eine Druckgasbehälter (13) mindestens eine Kammer aufweist, die dafür konfiguriert ist, ein komprimiertes unter Druck stehendes Fluid bei einem Volumen V_{PF} [ml] und einem Druck P_{PF} [barg] einzuschließen, und wobei der mindestens eine Druckgasbehälter mindestens eine Öffnung (13A) aufweist;
(iii) mindestens ein hohles Durchstechelement (11); wobei das mindestens eine hohle Durchstechelement (11) durch mindestens ein scharfes Ende (11P) und ein zweites Ende gekennzeichnet ist; wobei das zweite Ende in Fluidverbindung mit dem mindestens einen Druckgasbehälter (13) steht und die mindestens eine Öffnung (13A) des mindestens einen Druckgasbehälters (13) abdichtet; wobei das erste und das zweite Ende durch mindestens ein Hohlrohr miteinander fluidverbunden sind;
wobei die mindestens eine Fluideinlassöffnung der mindestens einen Kapsel hinsichtlich ihrer Größe und Form dafür konfiguriert ist, eine abdichtende Verbindung mit dem mindestens einen spitzen Ende (11P) des mindestens einen hohlen Durchstechelements (11) einzugehen;
wobei die Betätigung des mindestens einen Druckgasbehälters (13) dazu konfiguriert ist, das Durchstechen der mindestens einen Kapsel zu ermöglichen, um die mindestens eine Fluideinlassöffnung mit Hilfe des mindestens einen scharfen Endes (11P) des Durchstechelements (11) bereitzustellen, und wobei das Drücken auf den mindestens einen Druckgasbehälter (13) zu einer Relativbewegung des zweiten Endes des mindestens einen hohlen Durchstechelements (11) aus seiner Position führt, so dass die Abdichtung der mindestens einen Öffnung (13A) des mindestens einen Druckgasbehälters aufgehoben wird, um es dem unter Druck stehenden Fluid zu ermöglichen, aus der mindestens einen Kammer auszutreten und durch ein Hohlrohr des mindestens einen hohlen Durchstechelements (11) in die mindestens eine Kapsel einzutreten, um die eine oder die mehreren Substanzen mitzureißen und diese in die mindestens eine Körperhöhle abzugeben.

2. Vorrichtung nach Anspruch 1, wobei die mindestens eine Kammer aus einem Material besteht, das eine Hochbarrierefolie ist; wobei die Hochbarrierefolie eine Aluminium-Hochbarrierefolie ist.

3. Vorrichtung nach Anspruch 1, wobei die Betätigung des mindestens einen Druckgasbehälters (13) das Drücken auf denselben umfasst.

4. Vorrichtung nach Anspruch 1, wobei das zweite Ende des mindestens einen hohlen Durchstechelements (11) und die mindestens eine Kammer als eine Einheit ausgebildet sind, sodass die Oberseite (35) der mindestens einen Kammer in einem Stück mit dem zweiten Ende des mindestens einen hohlen Durchstechelements (11) ausgebildet ist.

5. Vorrichtung nach Anspruch 1, wobei das mindestens eine scharfe Ende (11P) des mindestens einen hohlen Durchstechelements (11) mindestens eine zweite Öffnung aufweist, durch die das unter Druck stehende Fluid in die mindestens eine Kapsel eintritt.

6. Vorrichtung nach Anspruch 5, zusätzlich aufweisend mindestens ein Dichtungsseptum, das zum Umschließen und Abdichten der mindestens einen zweiten Öffnung in dem mindestens einen spitzen Ende (11P) des mindestens einen hohlen Durchstechelements (11) vor Betätigung des mindestens einen Druckgasbehälters (13) ausgelegt ist.

7. Vorrichtung nach Anspruch 6, wobei bei Betätigung der Vorrichtung das mindestens eine Dichtungsseptum entfernt wird, um den Austritt des unter Druck stehenden Fluids durch die mindestens eine zweite Öffnung in dem mindestens einen hohlen Durchstechelement (11) zu ermöglichen.

8. Vorrichtung nach Anspruch 7, wobei bei Betätigung der Vorrichtung der mindestens eine Druckgasbehälter (13) zusammengedrückt wird und das mindestens eine hohle Durchstechelement (11) zu dem mindestens einen Nasenelement (4) geschoben wird, sodass der unterste Teil des mindestens einen Nasenelements (4) das Dichtungsseptum von der Umrandung der mindestens einen zweiten Öffnung in dem mindestens einen spitzen Ende (11P) des mindestens einen hohlen Durchstechelements (11) entfernt.

9. Vorrichtung nach Anspruch 8, wobei das mindestens eine scharfe Ende (11P) des mindestens einen hohlen Durchstechelements (11) mindestens eine zweite Öffnung aufweist, sodass bei Betätigung der Vorrichtung das unter Druck stehende Fluid durch die mindestens eine zweite Öffnung des mindestens einen hohlen Durchstechelements (11) in die mindestens eine Kapsel eintritt.

10. Vorrichtung nach Anspruch 1, wobei die mindestens eine Kammer die mindestens eine Öffnung (13A) aufweist, durch die das unter Druck stehende Fluid aus der mindestens einen Kammer austritt; wobei ferner mindestens eine der folgenden Bedingungen erfüllt ist: (a) das zweite Ende des mindestens einen hohlen Durchstechelements (11) verschließt die mindestens eine Öffnung (13A) der mindestens einen Kammer vor Betätigung der Vorrichtung; (b) bei Betätigung der Vorrichtung wird die Abdichtung der mindestens einen Öffnung (13A) der mindestens einen Kammer aufgehoben, sodass das unter Druck stehende Fluid durch die mindestens eine Öffnung (13A) der mindestens einen Kammer in das Hohlrohr des mindestens einen hohlen Durchstechelements (11) austritt; (c) sowie eine beliebige Kombination davon.

11. Vorrichtung nach Anspruch 10, wobei das mindestens eine scharfe Ende (11P) des mindestens einen hohlen Durchstechelements (11) mindestens eine zweite Öffnung aufweist, sodass bei Betätigung der Vorrichtung das unter Druck stehende Fluid durch die mindestens eine zweite Öffnung des mindestens einen hohlen Durchstechelements (11) in die mindestens eine Kapsel eintritt.

12. Vorrichtung nach Anspruch 1, wobei mindestens eine der folgenden Bedingungen erfüllt ist:
(a) das mindestens eine Nasenelement (4) weist mindestens eine Abdeckung des Nasenelements (4) auf, die hinsichtlich Größe und Form zum zumindest teilweise abdichtenden Abdecken des mindestens einen Nasenelements (4) konfiguriert ist; und wobei die mindestens eine Abdeckung des Nasenelements und das mindestens eine Nasenelement (4) miteinander verbunden sind;
(b) das mindestens eine Nasenelement (4) mindestens eine Öffnung aufweist, durch die die mindestens eine Substanz aus der Vorrichtung austritt, sodass die mindestens eine Abdeckung des Nasenelements (4) die mindestens eine Öffnung abdichtet und deren Entfernen die Abdichtung aufhebt;
(c) wobei die mindestens eine Kapsel aus einer Gruppe ausgewählt ist, die aus einem durchstechbaren Behälter (2), einer "Blow-Fill"-Dichtung und einer "Form-Fill"-Dichtung sowie einer beliebigen Kombination davon besteht;
(d) die mindestens eine Kammer ist ein Behälter (2), der zum Halten des unter Druck stehenden Fluids für einen bestimmten Zeitraum bei dem Druck P_{PF} ausgelegt ist;
(e) die mindestens eine Kapsel besteht aus mindestens einem Material, ausgewählt aus einer Gruppe bestehend aus Polyethylen hoher oder niedriger Dichte, Polypropylen hoher oder niedriger Dichte, einem beliebigen Kunststoffharz, Glas und einer beliebigen Kombination davon;
(f) die mindestens eine Kammer zum Aufnehmen des Volumens V_{PF} [ml] des unter Druck stehenden Fluids bei einem Druck P_{PF} [barg] und dazu konfiguriert ist, dass das unter Druck stehende Fluid aus der mindestens einen Kammer innerhalb kurzer Zeit <500 Millisekunden (dT) über die mindestens eine Fluideinlassöffnung abgegeben wird, die Substanzen mitreißt und über die mindestens eine Fluidauslassöffnung in die mindestens eine Körperhöhle abgibt, sodass die Freisetzungszeit des Volumens V_{sub} [ml oder mg] der Substanzen und des V_{PF} [ml] des unter Druck stehenden Fluids, dTᵣₑₗₑₐₛₑ,weniger als 500 Millisekunden beträgt;
(g) eine beliebige Kombination davon.

13. Vorrichtung nach Anspruch 1, wobei die Vorrichtung dazu konfiguriert ist, die Substanz V_{sub} und das unter Druck stehende Fluid V_{PF} durch die mindestens eine Fluidauslassöffnung mit einem Durchmesser D [mm] abzugeben, wobei mindestens eine der folgenden Bedingungen erfüllt ist:
a. V_{PF} liegt in einem Bereich von 1 bis 50 ml;
b. V_{sub} liegt in einem Bereich von etwa 0,01 bis 7 ml;
c. Dᵢₙ und/oder Dₒᵤₜ liegt in einem Bereich von 0,2 bis 6 mm;
d. P_{PF} liegt in einem Bereich von etwa 0 bis 10 barg;
e. eine Druckgeschwindigkeit ist größer als 0,001 barg/ms;
f. die Druckgeschwindigkeit ist größer als 0,01 barg/ms;
g. eine Volumenrate dV_{sub}/dT oder dV_{sub}/dTᵣₑₗₑₐₛₑ ist größer als 0,0001 ml/ms;
h. die Volumenrate dV_{sub}/dT oder dV_{sub}/dTᵣₑₗₑₐₛₑ ist größer als 0,001 ml/ms;
i. die Volumenrate dV_{PF}/dT oder dV_{PF}/dTᵣₑₗₑₐₛₑ ist größer als 0,001 ml/ms;
j. die Volumenrate dV_{PF}/dT oder dV_{PF}/dTᵣₑₗₑₐₛₑ ist größer als 0,01 ml/ms;
k. eine beliebige Kombination davon.

14. Vorrichtung nach Anspruch 1, wobei mindestens eine der folgenden Bedingungen erfüllt ist:
a. die mindestens eine Körperhöhle ist ausgewählt aus einer Gruppe bestehend aus: einer Nasenhöhle, dem Mund, dem Rachen, einem Ohr, dem Auge, der Vagina, dem Rektum, der Urethra und einer beliebigen Kombination davon;
b. das unter Druck stehende Gas ist ausgewählt aus einer Gruppe bestehend aus Luft, Stickstoff, Sauerstoff, Kohlendioxid, Helium, Neon, Xenon, Stickstoffoxid und einer beliebigen Kombination davon;
c. während der Abgabe der mindestens einen Substanz bildet ein Gemisch des vorbestimmten Volumens V_{gas} [ml] des unter Druck stehenden Gases mit dem vorbestimmten Volumen V_{sub} [ml oder mg] der darin mitgerissenen Substanz eine Wolke von Aerosol (718); wobei das Aerosol (718) eine vorbestimmte Verteilung aufweist, wobei die Verteilung entweder homogen oder heterogen ist, die heterogene Verteilung ausgewählt ist aus einer Gruppe bestehend aus: einer willkürlichen Verteilung, einer Verteilung, bei der die Dichte der mindestens einen Substanz innerhalb des Gemischs einem vorbestimmten Muster folgt, und einer beliebigen Kombination davon; Eigenschaften des Aerosols (718) sind ausgewählt aus einer Gruppe bestehend aus: Teilchengröße, Teilchenform, Teilchenverteilung und einer beliebigen Kombination davon, sind bestimmbar aus Eigenschaften der Vorrichtung, ausgewählt aus einer Gruppe bestehend aus: dem vorbestimmten Volumen des unter Druck stehenden Gases, dem vorbestimmten Volumen der Substanz, dem vorbestimmten Druck des unter Druck stehenden Gases, der vorbestimmten Öffnungsgröße und einer beliebigen Kombination davon;
d. mindestens eine der Substanzen ist ausgewählt aus einer Gruppe bestehend aus einem Gas, einer Flüssigkeit, einem Pulver, einem Aerosol (718), einer Aufschlämmung, einem Gel, einer Suspension und einer beliebigen Kombination davon;
e. mindestens eine der Substanzen wird entweder unter einer inerten Atmosphäre oder unter Vakuum gelagert, um Reaktionen während der Lagerung zu verhindern;
f. eine Dosis-Wirkungs-Kurve für die Konzentration der Substanz im Gehirn bei nasaler Verabreichung über die Vorrichtung ist im Wesentlichen linear; und
g. eine Dosis-Wirkungs-Kurve für die Konzentration im Gehirn, deren Anpassungsfunktion aus einer Gruppe ausgewählt ist, die aus logarithmischen, parabolischen, exponentiellen, sigmoiden, Potenz-Low-Funktionen und beliebigen Kombinationen davon besteht; bei nasaler Verabreichung der Substanz über die Vorrichtung.

15. Vorrichtung nach Anspruch 1, wobei mindestens eine der folgenden Eigenschaften zutrifft:
(a) die mindestens eine Abdeckung des Nasenelements (4) ist zum Bilden eines luftdichten Verschlusses für die mindestens eine Öffnung konfiguriert, wobei die Öffnungsabdeckung entlang der Vorrichtung verschiebbar, um die Vorrichtung drehbar, um ein Scharnier an der Außenseite der Vorrichtung drehbar ist, sowie jede Kombination davon;
(b) die Vorrichtung zum pulsierenden Mitreißen der Substanz durch das unter Druck stehende Fluid konfiguriert ist, sodass mehrere Teile V_{PF} über die mindestens eine Fluidauslassöffnung in die mindestens eine Körperhöhle austreten;
(c) die Vorrichtung weist ferner eine Sicherheitsverriegelung (720) auf, die zum Verhindern einer versehentlichen Betätigung der Vorrichtung ausgelegt ist;
(d) die Substanz ist ausgewählt aus einer Gruppe bestehend aus Proteinen; Stammzellen; Zellen, Organen, Teilen, Extrakten und Isolaten davon; Makromolekülen; RNA oder anderen Genen und für Proteine codierenden Materialien; Neurotransmittern; Rezeptorantagonisten; Hormonen; Ketamin; dem von Lilly (USA) im Handel erhältlichen Produkt "Baqsimi"; Glucagon; Substraten zur Behandlung einer der folgenden Erkrankungen: Anaphylaxie, Parkinson, Krampfanfälle und Opioid-Überdosierung; Adrenalin; Atropin; Metoclopramid; im Handel erhältlichen Naloxon- oder Narcan-Produkten; Esketamin (Spravato); Radicava [Edaravon]; Ingrezza [Valbenazin]; Austedo [Deutetrabenazin]; Ocrevus [Ocrelizumab]; Xadago [Safinamid]; Spinraza [Nusinersen]; Zinbryta [Daclizumab]; Nuplazid [Pimavanserin]; Aristada [Aripiprazol-Lauroxil]; Vraylar [Cariprazin]; Rexulti [Brexpiprazol]; Aptiom [Eslicarbazepinacetat]; Vizamyl [Flutemetamol-F18-Injektion]; Brintellix [Vortioxetin]; Tecfidera [Dimethylfumarat]; Dotarem [Gadoterat-Meglumin]; antikörpervermittelten, auf das Gehirn ausgerichteten Wirkstoffabgaben, darunter Aducanumab, Gantenerumab, Bapineuzumab, Solanezumab, Ofatumumab CD20, BIIB033, LCN2, HMGB 1; Insulin; Oxytocin; Orexin-A; Leptin; Benzodiazepin, Midazolam; Naloxon; Perillylalkohol; Camptothecin; sekundären Pflanzenstoffen, darunter Curcumin und Chrysin; Nukleotiden; Olanzapin; Risperidon; Venlafaxin; GDF-5; Zonisamid; Ropinirol; pflanzlichen und synthetisch hergestellten Terpenen und Cannabinoiden, darunter THC und CBD; Valproinsäure; Rivastigmin; Östradiol; Topiramat oder ein gleichwertiges Präparat mit der CAS-Nr. 97240-79-4; MFSD2 oder MFSD2A oder der natriumabhängige Lysophosphatidylcholin-Symporter; Midazolam; Naloxon; Perillylalkohol; Camptothecin; sekundären Pflanzenstoffen, darunter Curcumin und Chrysin; Nukleotiden; Olanzapin; Risperidon; Venlafaxin; GDF-5; Zonisamid; Ropinirol; pflanzlichen und synthetisch hergestellten Terpenen und Cannabinoiden, darunter THC und CBD; Valproinsäure; Rivastigmin; Östradiol; Topiramat oder ein gleichwertiges Präparat mit der CAS-Nr. 97240-79-4; MFSD2 oder MFSD2A oder der natriumabhängige Lysophosphatidylcholin-Symporter; sowie alle Ester, Salze, Derivate, Mischungen und Kombinationen davon, mit oder ohne Trägerstoff, Liposomen, lipophile oder wassermischbare Lösungsmittel, Tenside, Zellen und Zellfraktionen in einer therapeutisch wirksamen Konzentration; (e) beliebige Kombinationen davon.

16. Vorrichtung nach Anspruch 1, wobei die mindestens eine Kapsel ein Hohlrohr (54) ist, das durch mindestens zwei miteinander verbundene Enden gekennzeichnet ist, von denen mindestens eines proximal zu der mindestens einen Kammer angeordnet ist.

17. Vorrichtung nach Anspruch 16, wobei mindestens eine der folgenden Bedingungen erfüllt ist:
(a) die mindestens eine Kapsel weist mindestens ein kugelförmiges Element auf, das an mindestens einem der Enden angeordnet ist und zum Abdichten der mindestens einen Kapsel und zum Verhindern eines Austretens des mindestens einen Substrats aus dieser ausgelegt ist;
(b) die mindestens eine Kapsel weist zwei kugelförmige Elemente auf, von denen jeweils eines an jedem der Enden angeordnet ist, sodass das mindestens eine Substrat dazwischen positioniert ist; wobei die beiden kugelförmigen Elemente bei Austreten des unter Druck stehenden Fluids aus der mindestens einen Kammer zum Vermischen des mindestens einen Substrats und des mindestens einen Substrats ausgelegt sind;
(c) die mindestens eine Kapsel weist mindestens eine Membran auf, die an mindestens einem der Enden angeordnet ist und zum Abdichten der mindestens einen Kapsel und zum Verhindern eines Austretens des mindestens einen Substrats daraus ausgelegt ist; wobei die mindestens eine Kapsel zwei Membranen aufweist, von denen jeweils eine an jedem der Enden angeordnet ist, sodass das mindestens eine Substrat dazwischen positioniert ist;
(d) die mindestens eine Kapsel weist mindestens ein Entenschnabelventil auf, das an mindestens einem der Enden angeordnet ist und zum Abdichten der mindestens einen Kapsel und zum Verhindern eines Austretens des mindestens einen Substrats daraus ausgelegt ist; wobei die mindestens eine Kapsel zwei Entenschnabelventile aufweist, von denen jeweils eines an jedem der Enden angeordnet ist, sodass das mindestens eine Substrat dazwischen positioniert ist;
(e) wobei die mindestens eine Kapsel mindestens ein kugelförmiges Element, eine Membran, ein Einwegventil, ein Entenschnabelventil und eine beliebige Kombination davon aufweist;
(f) eine beliebige Kombination davon.

## Revendications

1. Dispositif permettant d'administrer une ou plusieurs substances à l'intérieur d'au moins une cavité corporelle, comprenant :
i) au moins une pièce nasale (4) comprenant au moins une capsule comprenant V_{sub} [ml ou mg] desdites une ou plusieurs substances ; ladite au moins une capsule présentant au moins un orifice d'entrée de fluide de diamètre Dᵢₙ [mm] et au moins une sortie de décharge de fluide de diamètre Dₒᵤₜ [mm], configurée pour être placée à proximité de ladite au moins une cavité corporelle ;
ii) au moins un récipient de gaz sous pression (13) en communication avec ladite au moins une pièce nasale (4), ledit au moins un récipient de gaz sous pression (13) comprend au moins une chambre configurée pour confiner un fluide comprimé et pressurisé à un volume V_{PF} [ml] et une pression P_{PF} [barg] et dans lequel ledit au moins un récipient de gaz sous pression comprend au moins un orifice (13A) ;
iii) au moins un élément perforant creux (11) ; ledit au moins un élément perforant creux (11) est **caractérisé par** au moins une extrémité pointue (11P) et une deuxième extrémité ; ladite deuxième extrémité étant en communication fluidique avec ledit au moins un récipient de gaz sous pression (13) et scellant le au moins un orifice (13A) du au moins un récipient de gaz sous pression (13) ; lesdites première et deuxième extrémités sont interconnectées l'une à l'autre de manière fluidique par au moins un tube creux ;
ledit au moins un orifice d'entrée de fluide de ladite au moins une capsule est configuré, par sa taille et sa forme, pour s'interfacer de manière étanche avec ladite au moins une extrémité pointue (11P) dudit au moins un élément perforant creux (11) ;
dans lequel l'actionnement dudit au moins un récipient de gaz sous pression (13) est configuré pour permettre la perforation de ladite au moins une capsule afin de fournir ledit au moins un orifice d'entrée de fluide, au moyen de ladite au moins une extrémité pointue (11P) dudit élément perforant (11), et la pression exercée sur le au moins un récipient de gaz sous pression (13) entraîne un déplacement relatif de la deuxième extrémité du au moins un élément perforant creux (11) depuis sa position, de sorte que le scellement du au moins un orifice (13A) du au moins un récipient de gaz sous pression est supprimé, afin de permettre au fluide sous pression de sortir de ladite au moins une chambre et d'entrer, par un tube creux dudit au moins un élément perforant creux (11), dans ladite au moins une capsule, d'entraîner lesdites une ou plusieurs substances et de les délivrer dans ladite au moins une cavité corporelle.

2. Dispositif selon la revendication 1, dans lequel ladite au moins une chambre est constituée d'un matériau qui est un film à barrière élevée ; dans lequel ledit film à barrière élevée est un film d'aluminium à barrière élevée.

3. Dispositif selon la revendication 1, dans lequel l'actionnement dudit au moins un récipient de gaz sous pression (13) comprend la pression sur celui-ci.

4. Dispositif selon la revendication 1, dans lequel ladite deuxième extrémité dudit au moins un élément perforant creux (11) et ladite au moins une chambre sont formées en une seule unité, de sorte que la surface supérieure (35) de ladite au moins une chambre est intégrée à ladite deuxième extrémité dudit au moins un élément perforant creux (11).

5. Dispositif selon la revendication 1, dans lequel ladite au moins une extrémité pointue (11P) dudit au moins un élément perforant creux (11) comprend au moins un deuxième orifice à travers lequel ledit fluide sous pression pénètre dans ladite au moins une capsule.

6. Dispositif selon la revendication 5, comprenant en outre au moins un septum d'étanchéité, adapté, avant l'actionnement dudit au moins un récipient de gaz sous pression (13), pour entourer et sceller ledit au moins un deuxième orifice dans ladite au moins une extrémité pointue (11P) dudit au moins un élément perforant creux (11).

7. Dispositif selon la revendication 6, dans lequel, suite à l'actionnement dudit dispositif, ledit au moins un septum d'étanchéité est retiré pour permettre la sortie dudit fluide sous pression à travers ledit au moins un deuxième orifice dans ledit au moins un élément perforant creux (11).

8. Dispositif selon la revendication 7, dans lequel, suite à l'actionnement dudit dispositif, ledit au moins un récipient de gaz sous pression (13) est comprimé et ledit au moins un élément perforant creux (11) est poussé vers ladite au moins une pièce nasale (4) de sorte que la partie la plus basse de ladite au moins une pièce nasale (4) retire ledit septum d'étanchéité de la circonférence dudit au moins un deuxième orifice dans ladite au moins une extrémité pointue (11P) dudit au moins un élément perforant creux (11).

9. Dispositif selon la revendication 8, dans lequel ladite au moins une extrémité pointue (11P) dudit au moins un élément perforant creux (11) comprend au moins un deuxième orifice, de sorte qu'une fois ledit dispositif est actionné, ledit fluide sous pression pénètre dans ladite au moins une capsule à travers ledit au moins un deuxième orifice dudit au moins un élément perforant creux (11).

10. Dispositif selon la revendication 1, dans lequel ladite au moins une chambre comprend ledit au moins un orifice (13A) à travers lequel ledit fluide sous pression sort de ladite au moins une chambre ; dans lequel en outre au moins une des conditions suivantes est remplie : (a) ladite deuxième extrémité dudit au moins un élément perforant creux (11) scelle ledit au moins un orifice (13A) de ladite au moins une chambre, avant l'actionnement dudit dispositif ; (b) suite à l'actionnement dudit dispositif, ledit scellement dudit au moins un orifice (13A) de ladite au moins une chambre est supprimé, de sorte que ledit fluide sous pression sort à travers ledit au moins un orifice (13A) de ladite au moins une chambre dans ledit tube creux dudit au moins un élément perforant creux (11) ; (c) et l'une quelconque de leurs combinaisons.

11. Dispositif selon la revendication 10, dans lequel ladite au moins une extrémité pointue (11P) dudit au moins un élément perforant creux (11) comprend au moins un deuxième orifice, de sorte qu'une fois ledit dispositif est actionné, ledit fluide sous pression pénètre dans ladite au moins une capsule à travers ledit au moins un deuxième orifice dudit au moins un élément perforant creux (11).

12. Dispositif selon la revendication 1, dans lequel au moins une des conditions suivantes est remplie : (a) ladite au moins une pièce nasale (4) comprenant au moins un couvercle de pièce nasale (4) configuré, par sa taille et sa forme, pour recouvrir, de manière étanche, au moins partiellement ladite au moins une pièce nasale (4) ; et dans lequel ledit au moins un couvercle de pièce nasale et ladite au moins une pièce nasale (4) sont couplés l'un à l'autre ; (b) ladite au moins une pièce nasale (4) comprend au moins un orifice à travers lequel ladite au moins une substance sort dudit dispositif, de sorte que ledit au moins un couvercle de pièce nasale (4) scelle ledit au moins un orifice et que son retrait supprime ledit scellement ; (c) dans lequel ladite au moins une capsule est choisie dans un groupe constitué d'un récipient perçable (2), d'un récipient de type blow-fill-seal (BFS) et d'un récipient de type form-fill-seal (FFS) et l'une quelconque de leurs combinaisons ; (d) ladite au moins une chambre est un récipient (2) adapté pour contenir ledit fluide sous pression à ladite pression P_{PF} pendant une période de temps ; (e) ladite au moins une capsule est constituée d'au moins un matériau choisi dans un groupe constitué de polyéthylène haute ou basse densité, polypropylène haute ou basse densité, toute résine plastique, verre et l'une quelconque de leurs combinaisons ; (f) ladite au moins une chambre est configurée pour accepter ledit volume V_{PF} [ml] dudit fluide sous pression à la pression P_{PF} [barg] et configurée de sorte que ledit fluide sous pression soit libéré de ladite au moins une chambre dans un court laps de temps, <500 millisecondes (dT), via ledit au moins un orifice d'entrée de fluide, entraîne lesdites substances, jaillit via ledit au moins un orifice de sortie de décharge de fluide dans ladite au moins une cavité corporelle, de sorte que le temps de libération de ladite V_{sub} [ml ou mg] desdites substances et dudit V_{PF} [ml] dudit fluide sous pression, dTᵣₑₗₑₐₛₑ, soit inférieur à 500 millisecondes ; (g) l'une quelconque de leurs combinaisons.

13. Dispositif selon la revendication 1, dans lequel ledit dispositif est configuré pour délivrer ladite substance V_{sub} et le fluide sous pression V_{PF} à travers ledit au moins une sortie de décharge de fluide de diamètre D [mm], dans lequel au moins l'une des conditions suivantes est remplie :
a. V_{PF} est dans une plage de 1 à 50 ml ;
b. V_{sub} est dans une plage d'environ 0,01 à environ 7 ml ;
c. Dᵢₙ et/ou Dₒᵤₜ est dans une plage de 0,2 à 6 mm ;
d. P_{PF} est dans une plage d'environ 0 à environ 10 barg ;
e. une vitesse de pression est supérieure à 0,001 barg/ms ;
f. ladite vitesse de pression est supérieure à 0,01 barg/ms ;
g. un débit volumique dV_{sub}/dT ou dV_{sub}/dTᵣₑₗₑₐₛₑ est supérieur à 0,0001 ml/ms ;
h. ledit débit volumique dV_{sub}/dT ou dV_{sub}/dTᵣₑₗₑₐₛₑ est supérieur à 0,001 ml/ms ;
i. ledit débit volumique dV_{PF}/dT ou dV_{PF}/dTᵣₑₗₑₐₛₑ est supérieur à 0,001 ml/ms ;
j. ledit débit volumique dV_{PF}/dT ou dV_{PF}/dTᵣₑₗₑₐₛₑ est supérieur à 0,01 ml/ms ;
k. l'une quelconque de leurs combinaisons.

14. Dispositif selon la revendication 1, dans lequel au moins l'une des conditions suivantes est remplie :
a) ladite au moins une cavité corporelle est choisie dans un groupe constitué de la cavité nasale, la bouche, la gorge, une oreille, l'œil, le vagin, le rectum, l'urètre, et l'une quelconque de leurs combinaisons ;
b) ledit gaz sous pression est choisi dans un groupe constitué d'air, d'azote, d'oxygène, de dioxyde de carbone, d'hélium, de néon, de xénon, d'oxyde nitrique et l'une quelconque de leurs combinaisons ;
c) lors de la distribution de ladite au moins une substance, un mélange dudit volume V_{gas} [ml] prédéterminé dudit gaz sous pression avec ledit volume V_{sub} [ml ou mg] prédéterminé de ladite substance entraînée forme un panache d'aérosol (718) ; ledit aérosol (718) présentant une distribution prédéterminée, ladite distribution étant homogène ou hétérogène, ladite distribution hétérogène étant choisie dans un groupe constitué d'une distribution arbitraire, d'une distribution dans laquelle la densité de ladite au moins une substance dans ledit mélange suit un schéma prédéterminé, et l'une quelconque de leurs combinaisons ; les caractéristiques dudit aérosol (718) étant choisies dans un groupe constitué de : taille des particules, forme des particules, distribution des particules, et l'une quelconque de leurs combinaisons, peuvent être déterminées à partir des caractéristiques dudit dispositif choisies dans un groupe constitué de : ledit volume prédéterminé de gaz sous pression, ledit volume prédéterminé de ladite substance, ladite pression prédéterminée dudit gaz sous pression, ladite taille d'orifice prédéterminée, et l'une quelconque de leurs combinaisons ;
d) au moins une desdites substances est choisie dans un groupe constitué d'un gaz, d'un liquide, d'une poudre, d'un aérosol (718), d'une suspension, d'un gel, d'une suspension et l'une quelconque de leurs combinaisons ;
e) au moins l'une desdites substances est stockée soit sous atmosphère inerte, soit sous vide pour empêcher des réactions pendant le stockage ;
f) une courbe dose-réponse est sensiblement linéaire pour la concentration cérébrale de ladite substance lorsqu'elle est administrée par voie nasale via ledit dispositif ; et
g) une courbe dose-réponse pour la concentration cérébrale ayant un ajustement choisi dans un groupe constitué de logarithmique, parabolique, exponentiel, sigmoïde, loi de puissance, et l'une quelconque de leurs combinaisons ; de ladite substance lorsqu'elle est administrée par voie nasale via ledit dispositif.

15. Dispositif selon la revendication 1, dans lequel au moins l'une des conditions suivantes est remplie : (a) ledit au moins un couvercle de pièce nasale (4) est configuré pour fournir une fermeture étanche à l'air pour ledit au moins un orifice, ledit couvercle d'orifice pouvant coulisser le long dudit dispositif, tourner autour dudit dispositif, tourner autour d'une charnière sur l'extérieur dudit dispositif et l'une quelconque de leurs combinaisons ; (b) ledit dispositif est configuré de sorte que ledit fluide sous pression entraîne ladite substance de manière pulsée, de sorte qu'une pluralité de portions V_{PF} jaillit via ladite au moins une de sortie de décharge de fluide dans ladite au moins une cavité corporelle ; (c) ledit dispositif comprend en outre un loquet de sécurité (720), adapté pour empêcher un fonctionnement accidentel dudit dispositif ; (d) ladite substance est choisie dans un groupe constitué de protéines ; cellules souches ; cellules, organes, portions, extraits et isolats de ceux-ci ; macromolécules ; ARN ou autres gènes et des matériaux codant des protéines ; neurotransmetteurs ; antagonistes des récepteurs ; hormones ; kétamine ; produit Baqsimi disponible dans le commerce par Lilly (US) ; glucagon ; substrats pour traiter l'un des éléments suivants : anaphylaxie, Parkinson, crises et surdosage d'opioïdes ; épinéphrine ; atropine ; métoclopramide ; produits Naloxone ou Narcan disponibles dans le commerce ; Eskétamine (Spravato) ; Radicava [edaravone] ; Ingrezza [valbenazine] ; Austedo [deutetrabenazine] ; Ocrevus [ocrelizumab] ; Xadago [safinamide] ; Spinraza [nusinersen] ; Zinbryta [daclizumab] ; Nuplazid [pimavanserin] ; Aristada [aripiprazole lauroxil] ; Vraylar [cariprazine] ; Rexulti [brexpiprazole] ; Aptiom [eslicarbazépine acétate] ; Vizamyl [flutémétamol F18 injectable] ; Brintellix [vortioxétine] ; Tecfidera [fumarate de diméthyle] ; Dotarem [gadotérate de meglumine] ; administration de médicaments ciblant le cerveau médiée par des anticorps, y compris aducanumab, gantenerumab, bapineuzumab, solanezumab, ofatumumab CD20, BIIB033, LCN2, HMGB1 ; insuline ; ocytocine ; orexine-A ; leptine ; benzodiazépine, midazolam ; naloxone ; alcool périllylique ; camptothécine ; phytocomposés incluant curcumine et chrysin ; nucléotides ; olanzapine ; rispéridone ; venlafaxine ; GDF-5 ; zonisamide ; ropinirole ; terpènes et cannabinoïdes d'origine végétale et produits synthétiquement, y compris THC et CBD ; acide valproïque ; rivastigmine ; estradiol ; topiramate ou une préparation équivalente comprenant le n° CAS 97240-79-4 ; MFSD2 ou MFSD2A ou symporteur sodium-dépendant de lysophosphatidylcholine, midazolam ; naloxone ; alcool périllylique ; camptothécine ; phytocomposés incluant curcumine et chrysin ; nucléotides ; olanzapine ; rispéridone ; Venlafaxine ; GDF-5 ; zonisamide ; ropinirole ; terpènes et cannabinoïdes d'origine végétale et produits synthétiquement, y compris THC et CBD ; acide valproïque ; rivastigmine ; estradiol ; topiramate ou une préparation équivalente comprenant le n° CAS 97240-79-4, MFSD2 ou MFSD2A ou symporteur sodium-dépendant de lysophosphatidylcholine ; et tout ester, sel, dérivé, mélange, leurs combinaison, avec ou sans excipient, liposomes, solvants lyophiles ou miscibles à l'eau, tensioactifs, cellules, fractions cellulaires, à une concentration thérapeutiquement efficace ; (e) l'une quelconque de leurs combinaisons.

16. Dispositif selon la revendication 1, dans lequel ladite au moins une capsule est un tube creux (54) **caractérisé par** au moins deux extrémités interconnectées l'une à l'autre, dont au moins une est positionnée de manière proximale à ladite au moins une chambre.

17. Dispositif selon la revendication 16, dans lequel au moins l'une des conditions suivantes est remplie : (a) ladite au moins une capsule comprend au moins un élément sphérique positionné sur au moins l'une desdites extrémités, adapté pour sceller ladite au moins une capsule et pour empêcher toute fuite dudit au moins un substrat de celle-ci ; (b) ladite au moins une capsule comprend deux éléments sphériques, dont chacun est disposé à chacune desdites extrémités, de sorte que ledit au moins un substrat est positionné entre eux ; dans lequel lesdits deux éléments sphériques, une fois que ledit fluide sous pression sort de ladite au moins une chambre, sont adaptés pour mélanger ledit au moins un substrat et ledit au moins un substrat ; (c) ladite au moins une capsule comprend au moins une membrane positionnée sur au moins l'une desdites extrémités, adaptée pour sceller ladite au moins une capsule et pour empêcher toute fuite dudit au moins un substrat de celle-ci ; dans lequel ladite au moins une capsule comprend deux membranes, dont chacune est disposée à chacune desdites extrémités, de sorte que ledit au moins un substrat est positionné entre elles ; (d) ladite au moins une capsule comprend au moins une valve à bec de canard positionnée sur au moins l'une desdites extrémités, adaptée pour sceller ladite au moins une capsule et pour empêcher toute fuite dudit au moins un substrat de celle-ci ; dans lequel ladite au moins une capsule comprend deux valves à bec de canard, dont chacune est disposée à chacune desdites extrémités, de sorte que ledit au moins un substrat est positionné entre elles ; (e) dans lequel ladite au moins une capsule comprend au moins un élément sphérique, une membrane, une valve unidirectionnelle, une valve à bec de canard et l'une quelconque de leurs combinaisons ; (f) l'une quelconque de leurs combinaisons.
